(19) Europäisches Patentamt
European Patent Office
Office européen des brevets

(11) Publication number : **0 465 219 A1**

(12) **EUROPEAN PATENT APPLICATION**

(21) Application number : **91305991.1**

(22) Date of filing : **02.07.91**

(51) Int. Cl.$^5$ : **C07C 235/52,** C07C 235/74, C07C 235/12, C07C 275/16, C07C 237/12, C07F 7/18, B01D 71/06, B05D 1/20

(30) Priority : **02.07.90 JP 174968/90**
**02.07.90 JP 174969/90**
**28.03.91 JP 103728/91**

(43) Date of publication of application :
**08.01.92 Bulletin 92/02**

(84) Designated Contracting States :
**DE FR GB**

(71) Applicant : **Research Development Corporation of Japan**
**5-2, Nagatacho 2-chome**
**Chiyoda-ku Tokyo (JP)**
Applicant : **Itami, Yasuo**
**Koopo Okamoto 202, 1567, Aikawa-cho**
**Kurume-shi, Fukuoka (JP)**
Applicant : **Shimizu, Ryuichi**
**Kooporasu Sakai 305, 1623-6, Yamakawa-cho**
**Kurume-shi, Fukuoka (JP)**

(72) Inventor : **Kunitake, Toyoki**
**1-19-3, Sakuraoka, Shime-cho**
**Kasuya-gun, Fukuoka (JP)**
Inventor : **Takeda, Yoshiyuki**
**Suterahiruzu-nawate 207, s-8, Chuo-cho**
**Kurume-shi, Fukuoka (JP)**
Inventor : **Watanabe, Kyoko**
**19-22, Yakuin 2-chome, Chuo-ku**
**Fukuoka-shi, Fukuoka (JP)**
Inventor : **Itami, Yasuo**
**Ioopo Okamoto 202, 1567, Aikawa-cho**
**Kurume-shi, Fukuoka (JP)**
Inventor : **Shimizu, Ryuichi**
**22-201, 44-3, Yamaki, Ichihara-shi**
**Chiba-ken (JP)**

(74) Representative : **BATCHELLOR, KIRK & CO.**
**2 Pear Tree Court Farringdon Road**
**London EC1R 0DS (GB)**

(54) **Polyfluoroalkyl compounds, their production and uses.**

(57) Polyfluoroalkyl compounds :

$$(R\text{-}X\text{-})_2\text{-}Q\text{-}Y$$

wherein

R represents $CF_3(CF_2)_n$- (n : an integer of 7 to 13),

X represents a hydrocarbyl linking group containing 6 to 11 carbon atoms and optionally containing a double bond, triple bond or ether bond at any position,

Q represents a connector between the hydrophobic group of R-X- and the hydrophilic group of Y, and

Y represents a hydrophilic group.

The compounds may be produced by subjecting R-X-OH and L-glutamic acid to azeotropic dehydration reaction in the presence of a salt-forming agent of AH (wherein A represents, for example, a p-toluenesulfonate ion or a sulfate ion), subsequently an acylation reaction and a quaternizing reaction. The compounds may be used for forming a thin film by, for example, the Langmuir-Brodgett process. The film may be supported on a porous support to produce a membrane capable of separating substances.

EP 0 465 219 A1

FIG. 1

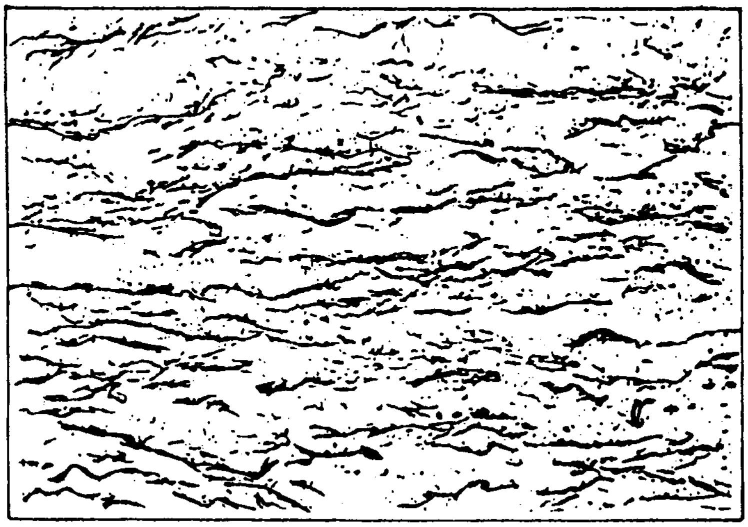

This invention is concerned with novel polyfluoroalkyl compounds, functional thin films formed from them, processes for their production and products which include such films.

As processes for producing thin films having regular molecular orientation, there have been known, for example, the Langmuir-Brodgett process (hereinafter abbreviated as LB process), a polymer-casting process and a process of casting a synthetic lipid to form a bimolecular film.

According to the LB process, a thin film is produced by preparing a solution of a given compound in an organic solvent, spreading the solution over a subphase, for example, water to form a monomolecular layer, then depositing the monomolecular layer on a proper substrate such as a glass plate, as is described in, for example, Keiji Iriyama; "Surface Film Molecular Design Series 1: Molecular Design of LB Film" (published by Kyoritu Shuppan K. K. on July 1, 1988), pp. 37-42. The LB film has a regularly oriented structure at a molecular level since the hydrophilic group of the compound is located against the subphase and, therefore, the molecules of the compound are oriented in the same direction with respect to molecular axis.

According to the polymer-casting process, a solution of a high molecular substance in a suitable solvent is cast in a film state, then the solvent is evaporated to produce a film, as is described in, for example, "Shin Jikken Kagaku Koza, vol. 19: High Polymer Chemistry" (published by Maruzen K. K.), pp. 971-980. The film produced by this process, however, has a complicated structure wherein regularly crystalline regions and non-crystaline regions coexist, with the crystalline regions being oriented in different directions.

According to the process of synthetic bimolecular film casting, a synthetic lipid capable of forming bimolecular film is dispersed in water and casting the dispersion on the surface of a substrate. This process enables one to easily obtain a thin film having a regular vectorial orientation of molecules.

The thus-obtained thin films are expected to be used as water- and oil-repelling surface-treating agents, surface lubricants, releasing agents, etc. because of their low surface activity.

As water- and oil-repelling surface-treating agents, silicone or fluorine-containing polymer coats have so far been used. Monomolecular layers of certain kinds of amphiphilic compounds having a long alkyl or fluoroalkyl chain or chains are also known to make a hydrophilic surface water-repellent. In some cases, a monomolecular layer of an amphiphilic compound having a group or groups capable of binding to the surface of a substrate is formed on the substrate. The thin film formed on the substrate gives the substrate surface water-repelling and oil-repelling properties and, as a result, the film prevents water and oils from depositing on the substrate and prevents generation of rust or stain, thus serving to maintain the substrate in a good state over a long period of time. In addition, in the case of giving lubricating properties or releasing properties to the surface, too, a similar thin film is formed on the surface of the substrate.

However, more reduction in thickness of the polymer coat for the purpose of increasing adaptability thereof to various devices leads to deterioration of film strength and uniformity. As a result, durability of the film is so deteriorated that it peels off from the substrate or is cracked, thus failing to provide intended functions.

On the other hand, the monomolecular layer of an amphiphilic compound having a group or groups capable of binding to the surface of the substrate, which is an ultra-thin film closely adhered to the surface of substrate, suffers no restrictions by dimension or shape of the substrate and has, therefore, a higher adaptability than the polymer coat. In some cases, however, introduction of the binding croup might damage the surface properties such as water- and oil-repelling properties, lubricating properties, releasing properties, etc. which the monomolecular layer essentially possesses. In addition, the introduced binding group is effective only for a specific surface of substrate, and hence various compounds must be prepared for various surfaces, resulting in less production flexibility. Furthermore, in case where a comparatively thick film is required, this type films are not suitable.

The thin films described above are also useful as films capable of separating a substance with high efficiency because of their special structure. For example, there have so far been proposed various separation films having selective gas permeability which are used for selectively obtaining a specific gas component to thereby enrich the gas component. In apparatuses utilizing combustion energy such as heaters, engines for automobiles and boilers, use of, for example, the air having an oxygen content enriched by this separation film serves to not only solve the problem of environmental pollution due to incomplete combustion, etc. but improve combustion efficiency itself.

As this type separation films, there have so far been used silicone rubbers. However, silicone rubbers, which have a high permeability coefficient necessary as selectively permeable film, have a low separation coefficient. Thus, development of film materials having both excellent permeability and separation coefficients have been made.

An amount of gas permeating through a uniform film is generally represented by the following formula:

$$R = (P \times \Delta p \times A)/\ell$$

wherein R represents a permeating rate of a gas [cc(STP)/sec], P represents permeation coefficient of the gas [cc(STP)·cm/cm$^2$·sec·cmHg], $\Delta p$ represents difference in partial pressure between the two sides of the film

[cmHg], A represents film area [$cm^2$], and $\ell$ represents film thickness [cm].

Accordingly, permeation rate, R, can be made large by reducing the thickness of the permeation film to the utmost without changing permeability coefficient, P, of the film material. Therefore, development of film materials having a somewhat satisfactory permeability coefficient and yet having a high separation coefficient and being capable of easily forming an ultra-thin film has been made. Processes for forming such ultra-thin film are also being studied.

For example, Japanese Unexamined Patent Publication Nos. 57-71605, 51-89564, etc. describe a gas-separating film wherein an ultra-thin film of an organic polymer prepared by the process of casting polymer over water surface is supported on a porous support. Japanese Unexamined Patent Publication No. 62-74406 proposes a selectively permeating thin film formed on a porous support by the interfacial reaction between a polyamine compound and a siloxane-containing isocyanate compound.

However, these conventional gas-separating films formed on a support have a practical thickness of about 0.1 μm at the thinnest. Films with a smaller thickness prepared by the conventional processes would have such a low mechanical strength that it can not maintain its shape. In addition, thin films with a uniform thickness becomes difficult to form and there result thin films having such deficiency as pinholes. If such thin film were formed with good accuracy by an improved filming process, it would not stably exist with keeping its shape when the thickness is reduced as thin as a molecular level since conventional materials have insufficient molecular orientation.

On the other hand, certain types of amphiphilic compounds are known to stably exist as monomolecular or bimolecular layer formed as a result of self-organizing regular orientation at a molecular level. Typical examples thereof are biolipids which form a bimolecular film including functional proteins and exert highly selective permeation in vivo. It is also known that a bimolecular lipid film artificially formed within fine pores shows selective permeation for a solute in an aqueous solution. This type films solve the problems of insufficient mechanical strength or insufficient accuracy in filming, which arise with other thin films, since the amphiphilic compounds themselves have film-forming ability.

Based on the above-described knowledge is obtained an oxygen-separating film allowing permeation at a high rate, by forming the film of a compound analogous to the biolipid and showing a highly selective permeability for a gas, such as a polyfluoroalkyl diethanolamine compound showing a high oxygen permeability, on a porous support by the LB process which is one of the processes for forming an organic ultra-thin film. (See "Polym. J.", Vol. 19, No. 2, p. 289 (1987) and "Koubunshi Ronbun-shu (Papers on High Polymers)", Vol. 43, No. 11, p. 761 (1986).)

However, the LB films formed of this type compounds have the defect that, as a monomolecular layer, they lack enough uniformity to function as substance-separating films. Therefore, in order to obtain a practically sufficient oxyge-separating ability, it is believed to be necessary to accumulate about 76 molecular layers, thickness of which is therefore not less than the conventional ones formed by other processes. The LB film generally has a high crystallinity, and it has been attempted to develop a technique of forming a defect-free film made of single crystals. However, such technique has not yet been established, and hence LB films so far formed are usually an assembly of fine crystal domains including many defects. Such defects are liable to act as pinholes for a gas. In addition, since a gas scarcely permeates through crystals with high density, it mainly permeates through the defects, resulting in no selective permeation.

Namely, conventional gas-separating films formed by using compounds analogous to biolipids are liable to form film defects since they are not in a highly self-organizing state and, in order to compensate for the defects, multilayer accumulation of 50 or more layers is believed to be necessary. As a result, an increase in thickness is inevitable.

Films formed by the LB process has a highly regular molecular orientation. However, the filming technique requires a high skill and involves difficult procedures. In addition, since water is generally used as sub-phase, the process can not be applied to a system involving a substance active or unstable for water.

The polymer-casting process has the defect that, though filming procedure itself is easy, films formed by this process have a low degree of molecular orientation. Therefore, functional members using this film have less reliability due to the insufficient orientation of the film.

On the other hand, thin films formed by casting a synthetic lipid capable of forming a biomolecular film possess an excellent regularity in molecular orientation and can be formed comparatively easily. However, since water is generally used as a solvent, this technique can not be applied to a system which contains a substance active or unstable for water or a system which contains a substance slightly soluble in water or soluble only in a specific solvent.

Fluorocarbon chains, which have a higher hydrophobicity than hydrocarbon chains, show excellent water- and oil-repelling properties and therefore have a small affinity for both organic solvents and water. Thus, fluorocarbon chains are advantageous for the use of the present invention. However, their molecular orientation

might be disturbed in comparison with hydrocarbon chains due to the rigidity and steric bulkiness of the fluorocarbon chains.

## SUMMARY OF THE INVENTION

An object of the present invention is to provide novel lipids which permit the use of various solvents and which enable one to produce various thin films, as a result of molecular design of skillfully combining the highly hydrophobic fluorocarbon chain and the hydrocarbon chain having a higher molecular orientation.

Another object of the present invention is to provide a thin film having excellent surface properties with no limitation as to dimension, shape, material, etc. of the substrate, by using a polyfluoroalkyl compound capable of forming a thin film having a regular molecular orientation in a self-organizing manner.

A further object of the present invention is to provide a substance-separating membrane having a defect-free, uniform molecular thin film composed of 1 to 50 monomolecular layers and having excellent permeation coefficient and separation coefficient.

A still further object of the present invention is to provide a process for preparing the polyfluoroalkyl compounds to be used for forming the aforesaid thin film in high purity and with good efficiency.

These and other objects of the present invention will become apparent from the following description thereof.

As a result of intensive inventigations, the inventors have found that the above-described and other objects of the present invention are attained by the synthetic lipids wherein a fluoroalkyl group and a hydrocarbyl group having sufficient chain length are linked to each other in the hydrophobic moiety thereof, and which can be cast over various solvents as well as water, thus having completed the present invention based on the findings.

That is, the polyfluoroalkyl compounds of the present invention are those represented by the following general formula:

$$(R\text{-}X\text{-})_2\text{-}Q\text{-}Y$$

wherein

R represents $CF_3(CF_2)_n$- (n: an integer of 7 to 13),

X represents a hydrocarbyl linking group containing 1 to 11 carbon atoms and optionally containing a double bond, triple bond or ether bond at any position,

Q represents a connector between the hydrophobic group of R-X- and the hydrophilic group of Y, and

Y represents a hydrophilic group.

Preferable examples of the compounds are those shown below wherein the connector, Q, in the above general formula, is a glutamic acid-containing residue.

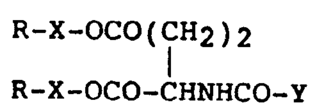

wherein

R represents $CF_3(CF_2)_n$- (n: an integer of 7 to 13),

X represents a hydrocarbyl linking group containing 1 to 11 carbon atoms and optionally containing a double bond, triple bond or ether bond at any position,

Q represents a connector between the hydrophobic group of R-X- and the hydrophilic group of Y, and

Y represents a hydrophilic group.

As other examples than the glutamic acid residue, there are illustrated aspartic acid residue, diethanolamine residue, etc.

These compounds may be synthesized as follows.

Firstly, 2-equivalent weight of an alcohol, R-X-OH (I), and L-glutamic acid are subjected to dehydration condensation under heating in the presence of a catalyst such as p-toluenesulfonic acid or sulfuric acid to obtain an L-glutamic acid diester (II). During this reaction to be conducted in an inert solvent such as toluene, benzene, 1,2-dimethoxyethane or chlorobenzene, the reaction temperature is maintained at 60-120°C, preferably 70-120°C.

Then, the active hydrogen of the $-NH_2$ group in the compound (II) is easily converted to a proper hydrophilic group, Y, to obtain an intended polyfluoro compound. As substituents employed as the hydrophilic group, Y, there are illustrated a variety of ones such as cationic ones (e.g., quaternary ammonium salts, etc.), anionic ones (e.g., carboxylic acids, etc.), nonionic ones (e.g., polyethylene oxides, etc.) and amphoteric ones (e.g.,

betaine type ones, etc.). The convertion reaction is not particularly limited, and various reactions may be employed. One example of the synthetic route is illustrated below. In the following scheme, A represents a p-toluenesulfonate ion, a sulfate ion, etc.

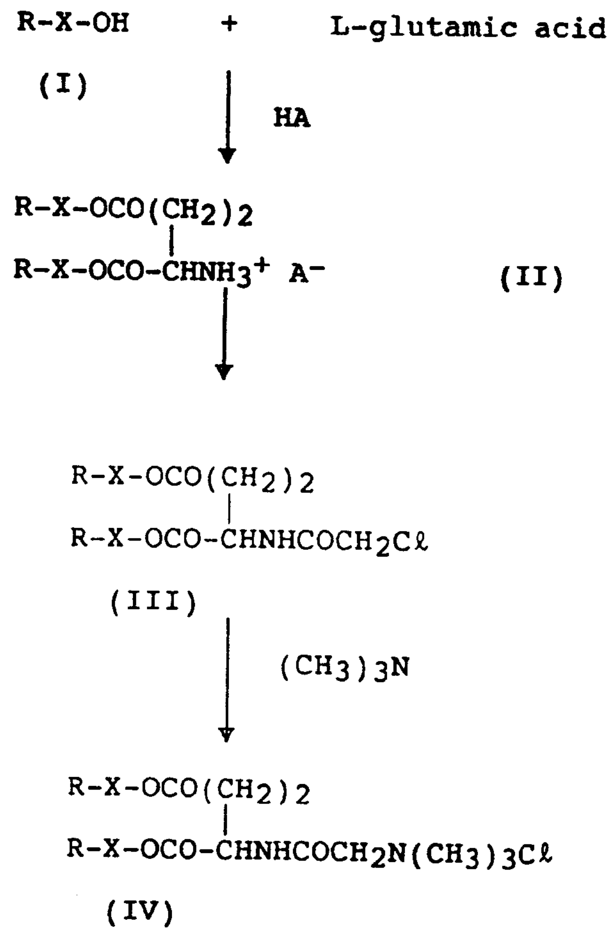


(I)

(II)

(III)

(IV)

Specific examples of the compound of the present invention are illustrated in Table 1. These compounds are identified through NMR spectrum, elemental analysis, etc. It is to be constructed, however, that Table 1 shows only illustrative specific examples, and does not limit the present invention at all.

Table 1

Examples of the polyfluoroalkyl compounds

| Compounds No. | Structural Formula |
| --- | --- |
| 1 | 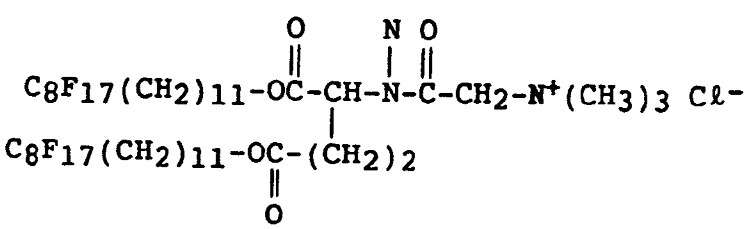 |

2

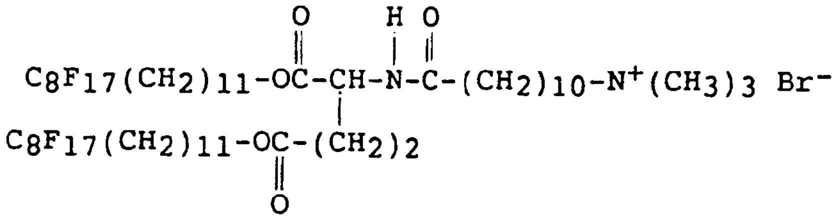

3

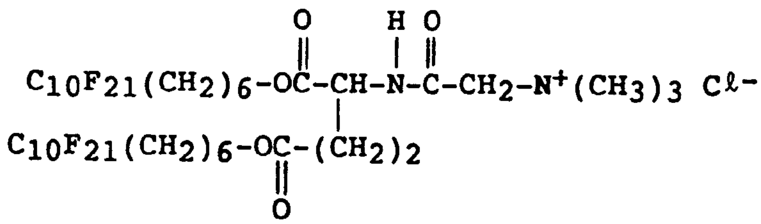

4

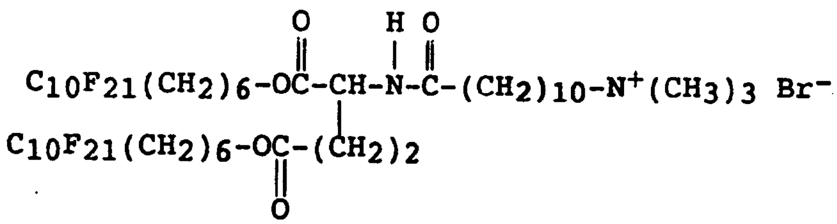

5

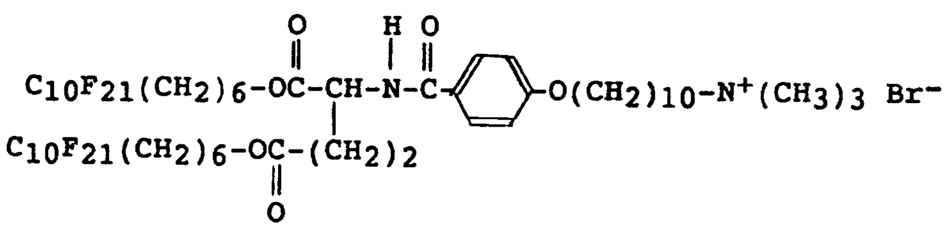

6

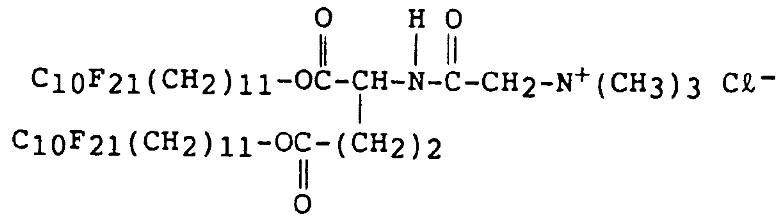

7

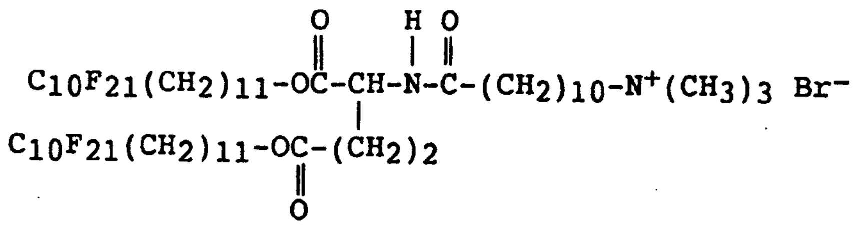

8

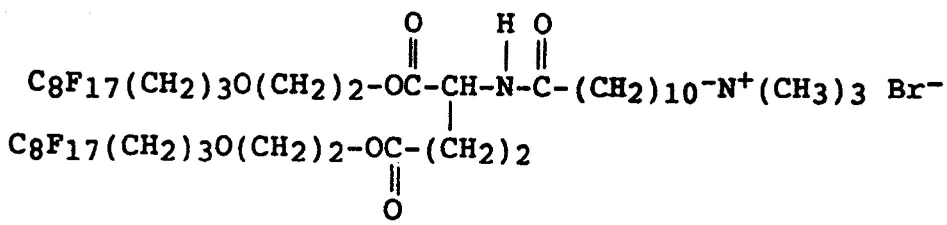

9

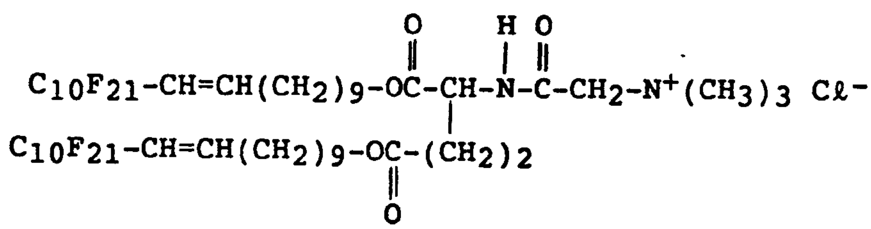

10

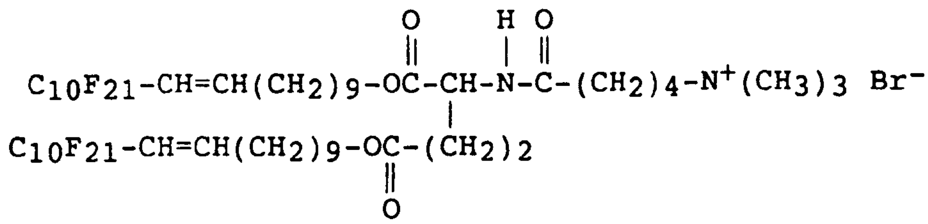

11

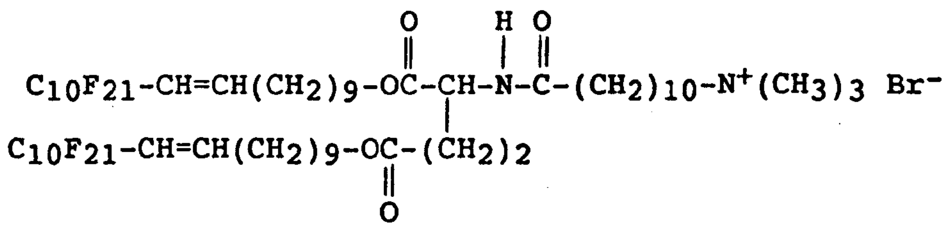

12

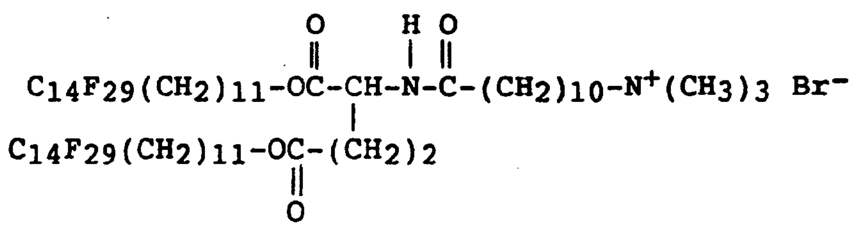

13

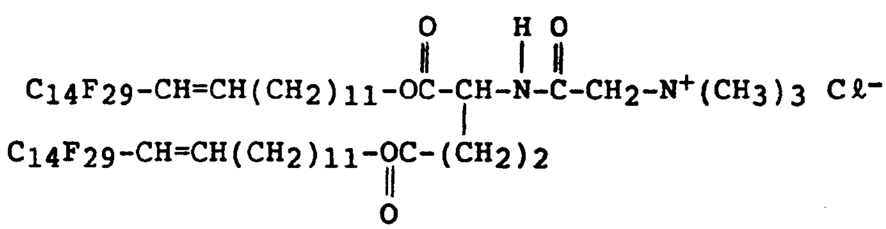

14

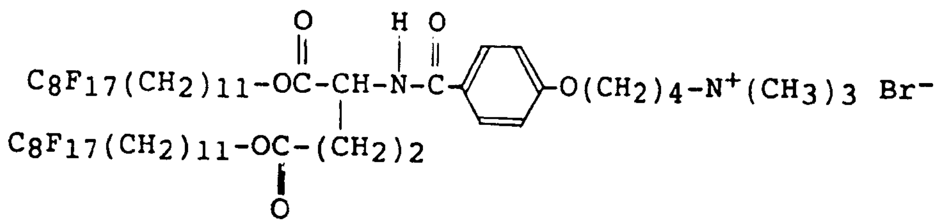

15

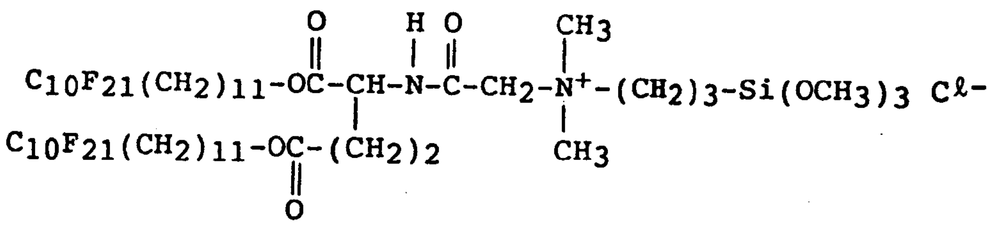

16

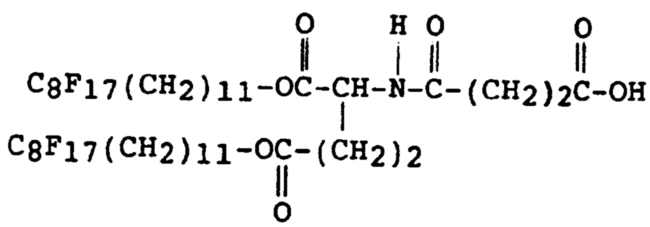

17

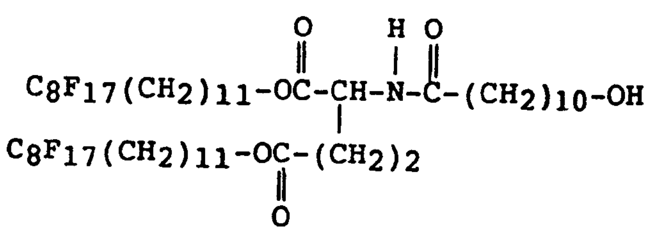

18

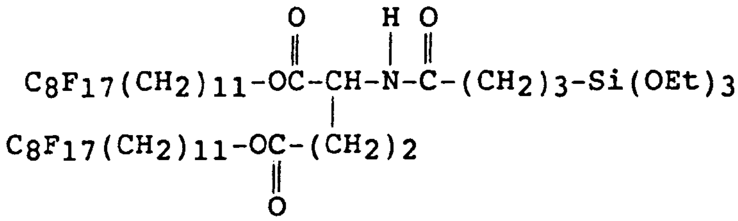

19

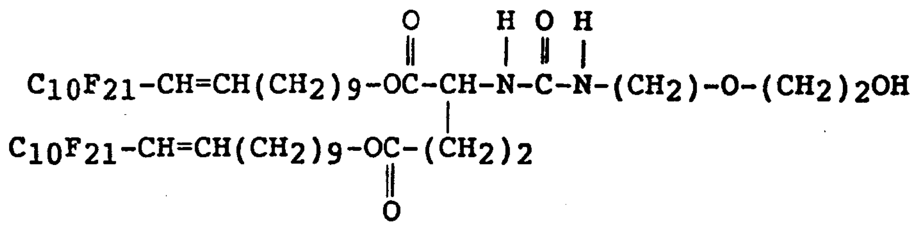

20

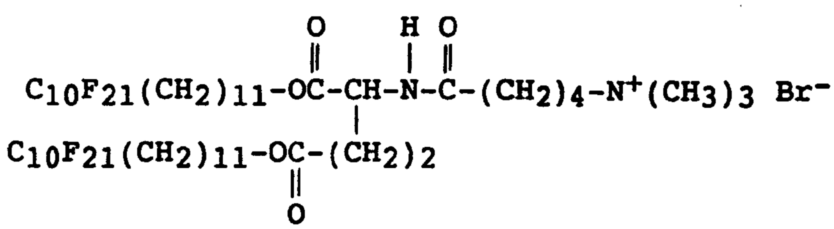

Cf.1

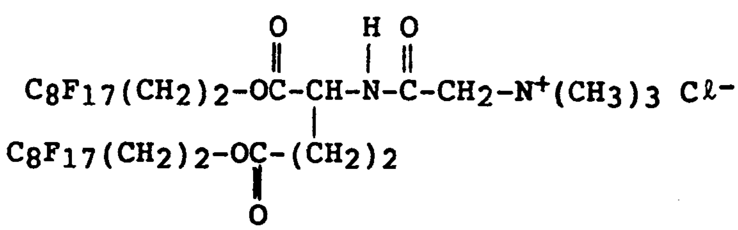

Cf. 2

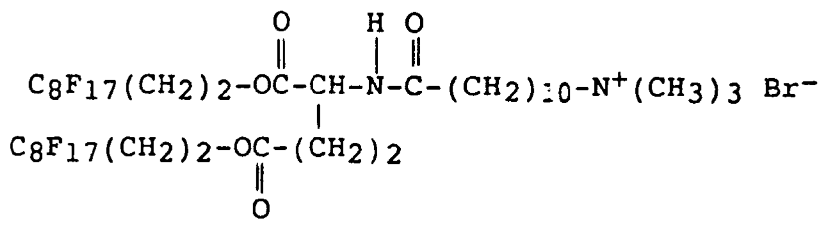

According to the above-described synthestic process, however, the yield of the end product is as low as 35-50% based on L-glutamic acid diester. This low yield is attributed to that, in case where acylation reaction is conducted in a polar solvent using a tertiary amine and an acylating agent, the yield of a resulting amido derivative is as low as 55-65% due to incomplete removal of the salt-forming agent. In addition, the quaternisation reaction rate is so slow that it takes 5 to 8 days for the reaction to complete under ordinary pressure. Thus, production efficiency of the process is quite low.

An improved synthesis scheme to be described below enables one to obtain the polyfluoroalkyl compounds with high purity in high yield.

That is, in synthesizing the polyfluoroalkyl compounds of the general formula (1):

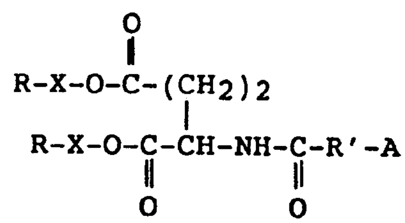

(1)

wherein

R represents $CF_3(CF_2)_n$- (n: an integer of 7 to 13),

X represents a hydrocarbyl linking group containing 6 to 11 carbon atoms and optionally containing a double bond, triple bond or ether bond at any position,

R′ represents a hydrocarbyl group containing 1 to 12 carbon atoms and optionally containing $-C_6H_4O-$ at any position, and

A represents a quaternary ammonium group, R-X-OH and L-glutamic acid are first reacted with each other with azeotropic dehydration in the presence of a salt-forming agent, AH (wherein A represents a p-toluenesulfonate ion or a sulfate ion). By this reaction is obtained a compound of the following formula (2):

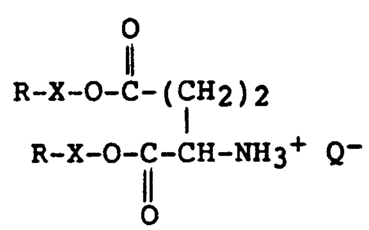

(2)

The compound (2) is then reacted with an alkali carbonate in a hydrophobic solvent of, for example, toluene or chlorobenzene to remove the salt-forming agent. The reaction solution is washed with water and, after separation of the aqueous phase, the compound (2) is reacted with a tertiary amine and an active derivative of ω-halocarboxylic acid to acylate. The thus acylated product contains a by-product in a content of several %. This by-product can be easily removed by contacting the acylated product in hydrophobic solution with an aqueous alkali carbonate solution at 50-90°C. The acylation process provides an amide derivative in a yield of 75-90% without decomposition.

The resulting amide derivative is then reacted with a tertiary amine such as trimethylamine to conduct quarternisation reaction. Conventional quarternisation processes involve the problem that, if the reaction is conducted under ordinary pressure, the reaction rate is too slow whereas, if conducted under high pressure condition, side reactions take place, resulting in production of the end product (1) with deteriorated quality. However, the end product (1) can be obtained with high purity by conducting the reaction at a temperature of 40-90°C under high pressure without any possible side reaction.

As is described above, the polyfluoroalkyl compounds of the present invention can be synthesized with high purity and in extremely high yield by conducting the acylation in a hydrophobic solvent, purifying the crude acylation product using a dilute sodium carbonate solution at 50-90°C, and adding thereto triethylamine to react for 3-10 hours under pressurized atmosphere. The thus-obtained polyfluoroalkyl compounds can be applied to both a system which contains a substance unstable to an aqueous or non-aqueous solvents and a system which contains a slightly water-soluble substance or soluble only in specific solvents, thus providing a wide choice of usable lipids. As a result, thin films with various properties and functions can be manufactured depending upon the end-use.

## BRIEF DESCRIPTION OF DRAWINGS

Fig. 1 is a scanning electron microscopic drawing showing a cross-section of the self-supporting cast film of synthetic lipid 9 obtained from ethylene glycol monomethyl ether in Example 9.

Fig. 2 is a more enlarged drawing thereof.

Fig. 3 is a similar drawing showing a cross-section of the self-supporting cast film of synthetic lipid 10 from perfluorohexane.

Fig. 4 is a more enlarged drawing thereof.

Fig. 5(a) is a diffraction pattern of the self-supporting cast film formed of synthetic lipid 10 using transmitted Cu-Ka ray, and Fig. 5(b) a diffraction pattern of the powder obtained by casting comparative compound Cf. 2 from perfluorohexane using transmitted Cu-Ka ray.

Figs. 6(a) to 6(s) respectively show IR charts of compounds No. 1 to No. 19.

## DETAILED DESCRIPTION OF THE PREFERRED EMBODIMENTS

In forming the thin film of the present invention using the compound of the invention, water is used as a solvent. In addition, organic solvents having a lower polarity than water, such as alcohols, ethers, ketones, esters, halogenated alkanes, nitriles, organic acids, organic bases, aromatic hydrocarbons, saturated or unsaturated hydrocarbons may be used as well. With lipids wherein the hydrophobic moiety is composed of only hydrocarbyl chain or chains, three-dimensional growth of crystal takes place if casting is conducted over the above-described organic solvent, thus no films being obtained.

With lipids, which contain a polyfluoroalkyl chain in the hydrophobic moiety but which have only a short hydrocarbyl chain between the fluorocarbon chain and the oxygen atom and are therefore rigid compounds, such as those compounds described in Japanese Unexamined Patent Publication No. 58-65256, growth of three-dimensional crystals is preferential to growth of bimolecular layer structure particularly in a solvent having a low polarity, though not so much serious as lipids wherein the hydrophobic moiety is composed of a hydrocarbyl chain alone. That is, the lipids can form the bimolecular layer structure only when the lipid concentration in the spreading solution is extremely low because of inhibition of growth of the bimolecular layer structure due to precipitation of fine crystals of the lipids only at a comparatively low concentration.

These defects have successfully been removed by introducing a long hydrocarbyl chain, a double bond, a triple bond, an ether bond, etc. between the fluorocarbon chain in the hydrophobic moiety and the oxygen atom as with the compounds of the present invention.

The compounds represented by the foregoing general formula of $(R\text{-}X)_2\text{-}Q\text{-}Y$ are compounds wherein a hydrocarbyl group having an enough length is bound to the highly hydrophobic fluoroalkyl group in the hydrophobic moiety. Introduction of the long hydrocarbon chain, double bond, triple bond, ether bond, etc. between the fluorocarbon chain, R, in the hydrophobic moiety and the connector moiety, Q, serves to give enough flexibility to the essentially rigid fluorocarbon chain. In addition, the long hydrocarbyl group also contributes to improvement of molecular orientation.

In producing a thin film using the compound of the present invention, the film-forming ability thereof is not essentially affected by the kind of employed filming process, since the uniform film-forming ability of the compound is based on its molecular structure. Therefore, a proper process adapted for a particular end-use may preferably be employed from among the various known filming processes.

For example, LB process has the advantage that it permits accurate control of thickness of the thin film, number of molecular layers, component ratio, density, etc. Although a limited number of compounds have been able to be formed into film by the LB process, the polyfluoroalkyl compounds of the present invention can be

formed into film by the LB process. That is, 1-to 50-layer LB accumulative film is obtained by spreading a solution of the polyfluoroalkyl compound in a suitable solvent on pure water retained in a trough for the LB process, two-dimensionally compressing the spread compound by means of, for example, a teflon bar to form a stable monomolecular layer composed of regularly oriented molecules of the compound on the surface of water, transferring the monomolecular layer onto the surface of a support plate, and optionally repeating these procedures.

With compounds having an ionic hydrophilic group such as compound 1 or 12 shown in Table 1, stability of the molecular layer may be increased by forming salt with a polyvalent ionic substance having a reverse charge. Alternatively, the lipid compounds of the present invention may be converted into salts before being spread on the surface of water or may be spread on the surface of water containing dissolved therein an excess amount of an ionic substance for forming salts in situ.

As the ionic substance, proper one is selected from among inorganic ions such as $Cd^{2+}$, $Ca^{2+}$, $Ba^{2+}$, $Mg^{2+}$, $A\ell^{3+}$, etc., ionic high molecular compounds such as carboxymethylcellulose, alginic acid, pectic acid, polyvinylsulfonic acid, polystyrenesulfonic acid, heparin, etc. These ionic substances may be used alone or as a combination of two or more. A preferred combination is a combination of compound 12 and alginic acid.

In some uses, it is preferred to mix the lipid with a specific substance such as a substance carrier. The polyfluoro compounds of the present invention have such an extremely high ability of spontaneously forming a film that they can form a stable monomolecular layer having high molecular orientation even when mixed with other substance. For example, a pyridine coordination product of salcomine which is a complex between salen and cobalt, known to have an affinity to oxygen, can be mixed with the polyfluoro compound and spread on the surface of water to obtain a stable monomolecular layer.

In case where the lipid compound of the present invention is combined with a specific substance, there results change in crystallinity. This change in crystallinity can be easily detected through the expansion-contraction behavior in the two-dimensional compression process of the monomolecular layer on the water surface. Therefore, films with a suitable crystallinity and without defects can be easily formed. For example, if the monomolecular layer on the water surface has too much contraction properties, a compound having a more flexibility is employed whereas, if too much expansion properties, a rigid compound having a more crystallinity is employed. Such control can be conducted by selecting a proper group to be introduced between the fluorocarbon chain and the connector moiety in the hydrophobic moiety of the lipid compound, thus imposing no restrictions on other constituents of the film. For example, expansibility of the monomolecular layer composed of the compound 2, 9, 13 and 10 shown in Table 1 may be enhanced by elongation of the hydrocarbyl group, introduction of double bond or introduction of ether bond, with the enhancement degree being increased in this order.

In case where an adsorption process is employed for producing a thin film, a monomolecular layer can be formed on a supporting substrate with extreme ease and in a short time without any special device and technique. Thus, the adsorption process is a practically quite advantageous film-forming process. The polyfluoro compounds of the present invention, which possess high ability of spontaneously filming properties, can be said to be substances suited for the adsorption process. Namely, monomolecular layer having regular orientation can be formed spontaneously merely by dipping a substrate into a dilute solution of the polyfluoro compound.

In addition, as described in Japanese Patent Application nos. 1-309923 and 2-59019, the polyfluoro compounds of the present invention are also suited for the casting process. This process is extremely advantageous from the practical point of view since molecule-accumulated films analogous to LB films can be easily formed.

The polyfluoro compounds, which possess a high ability of spontaneously forming a molecular layer, enable one to easily form a thin film wherein about 1 to about 1000 molecular layers are accumulated to form a regular layer structure. The thus-obtained thin film composed of the compound has a regular molecular orientation, and hence it has a markedly low energy surface characteristic of the oriented fluoroalkyl chain. In addition, structure of the surface molecular layer is not different between monomolecular layer and multimolecular film formed of a plurality of accumulated monomolecular layers. Accordingly, surface properties of the formed thin film are essentially independent from the number of molecular layers and thickness of the film as long as an ideal, defect-free molecular layers are formed.

Thus, an enough thin film can be formed depending upon requirements of particular devices for, for example, dimension and shape. Further, in case where the film is required to have a definite thickness for the purpose of obtaining enough durability or smoothing the rough surface of a substrate, a film with a predetermined thickness can be easily formed.

Additionally, even compounds which contain a polyfluoroalkyl chain in the hydrophobic moiety thereof and possess the ability of spontaneously forming monomolecular layer but which have only a short hydrocarbon chain between the fluorocarbon chain and the connector moiety and are therefore rigid are liable to generate fine defects in the film in comparison with the compounds of the present invention. As a result, such compounds show seriously decreased water-repelling properties when formed as an ultra-thin film composed of 1-10

molecular layers. According to the present invention, there is provided a thin film having excellent properties.

A substance-separating membrane having desired functions is obtained by supporting on a porous support an active layer composed of the selective thin film of the polyfluoroalkyl compound represented by the general formula of $(R\text{-}X)_2\text{-}Q\text{-}Y$.

The active layer in this substance-separating membrane is formed as a self-organizing monomolecular layer or, when a thicker film is suited for a particular purpose, as a film wherein the monomolecular layers are accumulated.

As film-forming processes, the LB process, adsorption process, etc. may be employed. Further, the thin film may also be formed according to one of the casting process, process of casting on water, interfacial reaction process, deposition process, etc.

In producing the thin film formed of the compound represented by the general formula of $(R\text{-}X)_2\text{-}Q\text{-}Y$, an adsorption process which is a practical and popular process for forming a monomolecular layer on a substrate may be employed in place of the LB process and the casting process, because the polyfluoro compounds have high self-organizing properties and spontaneously film-forming ability.

The porous support is not limited as to material as long as it can mechanically support the active layer to reinforce. As the material for the support, there are illustrated inorganic substances such as glass, metals, ceramics, etc. and organic polymer materials such as cellulose derivatives, polystyrene, polyvinylbutyral, polysulfone, polyvinyl chloride, polyesters, polyamides, polycarbonate, etc. It is particularly preferred to use those which have such structure that sizes of the surface pores range from about 10 to about 100 Å and that effective surface area of the pores is large enough or which have large internal voids and an asymmetric structure. That is, those which have pores of a molecular-level size on the surface and have a permeability as like as possible are preferred substrates. However, the substrates are not limited only to these. Pore sizes may be larger than the above-described range as long as the active layer has an enough strength. With respect to permeability, too, it suffices to employ a substrate having an enough permeability for the end-use of the film.

Additionally, as long as the self-organizing properties of the polyfluoroalkyl compounds are not damaged, polymers, crosslinking agents, plasticizers, oxygen carriers, ion carriers, etc. may be added to the thin film of active layers for the purpose of improving mechanical and chemical strength of the film or for accelerating migration of a permeable substance.

The group introduced between the fluorocarbon chain in the hydrophobic moiety and the connector moiety is selected taking into consideration crystallinity of the resulting film which depends upon length of the fluorocarbon chain and kinds of the hydrophilic group in the lipid, kinds of additives to the film, kinds of supporting substrate, kinds of a particular permeable substance and using conditions such as temperature. For example, lipids with too much flexibility fail to give preferable thin films since the films are in a liquid phase and have a decreased self-supporting ability.

Defects of the film can be prevented by properly selecting the introduced hydrocarbyl group for controlling flexibility. An ultra-thin film composed of only 1 to 50 molecular layers using the lipid having proper flexibility has high self-organizing and self-supporting properties and has no defects such as pinholes. Thus, the film shows excellent permeability and separating ability and is easy to handle. The thus-obtained thin film is used for separating various substances utilizing the excellent permeating properties. In particular, it is incorporated into a device for concentrating oxygen in the air utilizing the good affinity of the fluorocarbon to oxygen to thereby improve efficiency of combustion furnaces or engines or to treat patients suffering from respiratory diseases. In addition, the film can also be used for separating a gas such as hydrogen, carbon monoxide, carbon dioxide or sulfur dioxide. It can further be used for separating substances in a solution. Still further, since additives to be added to the film can freely be selected depending upon kind of a substance to be separated or environments in which it is used, it can find wide applications and are extremely advantageous.

The present invention is now illustrated in greater detail by reference to the following examples which, however, are not to be construed as limiting the present invention in any way.

(Synthesis of compounds)

Example I

Compound 5 was synthesized as follows.

18.9 g (30.4 mmols) of $C_{10}F_{21}(CH_2)_6OH$, 1.6 g (10.9 mmols) of L-glutamic acid, 2.8 g (14.7 mmols) of p-toluenesulfonic acid hydrate, and 250 ml of toluene were placed in a 500-ml flask, and the mixture was heated for 9 hours under reflux using a Dean-Stark trap with azeotropically removing water. After completion of the reaction, the reaction mixture as allowed to cool to room temperature, and solids were collected by filtration and washed with toluene. Recrystallization of the solids from ethanol yielded 6 g (7.6 mmols; yield: 70.1 %) of

a diester.

3.0 g (2.0 mmols) of the diester and 150 ml of tetrahydrofuran were placed in a 300-ml flask, and 1.0 g of triethylamine was added thereto under stirring. Then, a 20 ml solution of 1.0 g of p-(ω-bromodecanoxy) benzoyl chloride in tetrahydrofuran was dropwise added thereto over 20 minutes under ice-cooling. After stirring the mixture overnight, the solvent was distilled off, and the residue was dissolved in diethyl ether, followed by washing with water. The solution was then dried over anhydrous sodium sulfate. Diethyl ether was distilled off from the solution, and the residue was recrystallized from n-hexane to obtain 1.8 g (1.07 mmols; yield 54.2 %) of an amide.

100 ml of chloroform was placed in a 200-ml flask, 1.7 g (1.0 mmol) of the amide was dissolved therein, and 2 g of a trimethylamine gas was blown thereinto under stirring. After stirring the mixture for 7 days with tightly closing the flask, the solvent was distilled off, and the residue was recrystallized from n-hexane to obtain 1.1 g (0.63 mmol; yield: 63 %) of compound 5.

Example 2 (Synthesis of compound 9)

26.7 g (38.8 mmols) of $C_{10}F_{21}CH=CH(CH_2)_9OH$, 2.2 g (14.9 mmols) of L-glutamic acid, 3.9 g (20.5 mmols) of p-toluenesulfonic acid hydrate, and 300 ml of toluene were placed in a 500-ml flask, and the mixture was heated for 7 hours under reflux using a Dean-Stark trap with azeotropically removing water. After completion of the reaction, the reaction mixture was allowed to cool to room temperature, and solids were collected by filtration and washed with toluene. Recrystallization of the solids from ethanol yielded 13.4 g (8.1 mmols; yield: 54 %) of an diester.

3.0 g (2.0 mmols) of the diester and 100 ml of tetrahydrofuran were placed in a 200-ml flask, and 1.5 g of triethylamine was added thereto under stirring. Then, a 20 ml solution of 0.6 g (5.31 mmols) of chloroacetyl chloride in tetrahydrofuran was dropwise added thereto over 20 minutes under ice-cooling. After stirring the mixture overnight, the solvent was distilled off, and the residue was dissolved in diethyl ether, followed by washing with water. The solution was then dried over anhydrous sodium sulfate. Diethyl ether was distilled off from the solution, and the residue was recrystallized from n-hexane to obtain 1.8 g (1.15 mmols; yield: 57 %) of an amide.

100 ml of tetrahydrofuran was place in a 200-ml flask, 1.5 g of the amide was dissolved therein, and 3 g of a trimethylamine gas was blown thereinto under stirring. After stirring the mixture for 6 days with tightly closing the flask, the solvent was distilled off, and the residue was recrystallized from ethanol to obtain 1.3 g of compound 9.

Example 3

Compound 10 was synthesized as follows

26.7 g (38.8 mmols) of $C_{10}F_{21}CH=CH(CH_2)_9OH$, 2.2 g (15.0 mmols) of L-glutamic acid, 3.9 g (20.5 mmols) of p-toluenesulfonic acid hydrate, and 300 ml of toluene were placed in a 500-ml flask, and the mixture was heated for 7 hours under reflux using a Dean-Stark trap with azeotropically removing water. After completion of the reaction, the reaction mixture was allowed to cool to room temperature, and solids were collected by filtration and washed with toluene. Recrystallization of the solids from ethanol yielded 13.4 g (8.07 mmols; yield: 54 %) of a diester.

3.0 g (1.8 mmols) of the diester and 100 ml of tetrahydrofuran were placed in a 200-ml flask, and 0.8 g of triethylamine was added thereto under stirring. Then, a 20 ml solution of 1.0 g (5.0 mmols) of 5-bromopentanoyl chloride in tetrahydrofuran was dropwise added thereto over 20 minutes under ice-cooling. After stirring the mixture overnight, the solvent was distilled off, and the residue was dissolved in diethyl ether, followed by washing with water. The solution was then dried over anhydrous sodium sulfate. Diethyl ether was distilled off from the solution, and the residue was recrystallized from n-hexane to obtain 1.2 g (0.75 mmol; yield: 41.3 %) of an amide.

100 ml of tetrahydrofuran was placed in a 200-ml flask, 1.2 g (0.75 mmol) of the amide was dissolved therein, and 3 g of a trimethylamine gas was blown thereinto under stirring. After stirring the mixture for 6 days with tightly closing the flask, the solvent was distilled off, and the residue was recrystallized from ethanol to obtain 1.0 g (0.6 mmol; yield: 80 %) of compound 10.

Example 4 (Synthesis of compound 17)

28.8 g (48.78 mmols) of $C_8F_{17}(CH_2)_{11}OH$, 3.6 g (24.46 mmols) of L-glutamic acid, 6.0 g (31.5 mmols) of p-toluenesulfonic acid hydrate, and 300 ml of toluene were placed in a 500-ml flask, and the mixture was heated

for 7 hours under reflux using a Dean-Stark trap with azeotropically removing water. After completion of the reaction, the reaction mixture was allowed to cool to room temperature, and solids were collected by filtration and washed with toluene. Recrystallization of the solids from ethanol yielded 21.2 g (14.48 mmols; yield: 59.2%) of a diester.

1.46 g (1.0 mmol) of the diester and 20 ml of tetrahydrofuran were placed in a 200-ml flask, and 0.3 g of triethylamine was added thereto under stirring. Then, 0.32 g of diethyl cyanophosphate was added thereto under cooling with ice-water, and a 10 ml solution of 0.25 g (1.33 mmols) of ω-hydroxydecanoic acid in tetrahydrofuran was dropwise added thereto. After stirring the mixture overnight, the solvent was distilled off, and the residue was recrystallized from chloroform to obtain 1.0 g (0.68 mmols; yield: 68.4 %) of compound 17.

Example 5 (Synthesis of compound 19)

1.66 g of the diester obtained in Example 2 and 20 ml of tetrahydrofuran were placed in a 100-ml flask, 0.25 g of triethylamine was added thereto under stirring, and 0.1 g of triphosgene was further added thereto. Then, the mixture was refluxed for 2 hours. After allowing to cool, 1.5 g of aminoethoxyethanol was added thereto, followed by stirring the mixture for 2 hours at room temperature. After completion of the reaction, the residue was recrystallized from chloroform to obtain 1.1 g of compound 19.

Example 6 (Synthesis of compound 10; improvement of Example 3)

Synthesis of starting material:

23.5 g (34.1 mmols) of $C_{10}F_{21}CH{=}(CH_2)_9OH$, 2.06 g (14.0 mmols) of L-glutamic acid, 3.23 g (17.0 mmols) of p-toluenesulfonic acid hydrate and 70 ml of toluene were placed in a three-neck flask equipped with a stirrer, a Dean-Stark tap and a thermometer, and the mixture was heated under reflux for 7 hours with azeotropically removing produced water. After completion of the reaction, the reaction mixture was cooled to room temperature, and solids formed were collected by filtration. Recrystallization of the solids from toluene yielded 19.2 g (11.59 mmols; yield: 82.8 %) of a diester toluenesulfonate (hereinafter referred to as diester product).

Acylation reaction:

This diester product was added to 200 ml of toluene and 200 ml of a 10 % aqueous sodium carbonate solution, followed by stirring the mixture for 10 minutes at 50°C to remove p-toluenesulfonic acid. A part of toluene was azeotropically distilled off from the reaction solution for conducting dehydration. After cooling the solution, 1.52 g (15.0 mmols) of triethylamine and 2.54 g (12.75 mmols) of $Br(CH_2)_4COCl$ were added thereto, and the solution was heated to 40-50°C for 2 hours to react. Triethylamine hydrochloride precipitated after cooling was filtered off. Chromatographic analysis of solids in the thus-obtained filtrate revealed that the solids contained 93 % of an amide product and 7 % of a by-product.

In order to remove the by-product, the toluene solution was purified by adding thereto 100 ml of a 10 % aqueous solution of sodium carbonate and stirring the mixture at 80°C for 1 hour. Thereafter, the mixture was washed with water and dried by azeotropic distillation of toluene. Thus, 17.4 g (10.57 mmols; yield: 91.2 %) of an amide product (purity: 100 %) was obtained.

Quarternization reaction:

This amide product was added to a solution of 6.2 g (105 mmols) of triethylamine in 260 ml of tetrahydrofuran (THF), and the mixture was placed in an autoclave for 8 hours at 55-60°C to react. After completion of the reaction, THF was removed, and the residual solids were dissolved in chloroform. Insolubles were removed by filtration, and the solvent was removed. Recrystallization of the residue from n-hexane yielded 14.2 g (8.31 mmols) of compound 10. The yield was 78.6 %. Chromatographic analysis of the product revealed that purity of the product was 100 %.

Example 7 and 8

Compounds 9 and 15 were synthesized in the same manner as in Example 6 to obtain the following results.

Table 2

| Example No. | | | 7* | 8 |
|---|---|---|---|---|
| End compound | | | 9 | 20 |
| Acyl-ation | Diester product | g | 17.5 | 15.7 |
| | | mmol | 10.55 | 9.36 |
| | Purity of crude crystal (%) | | 91.5 | 92.2 |
| | Purified amide product | Purity (%) | 99.9 | 100 |
| | | Yield (%) | 85.2 | 75.6 |
| Quarter-nisation | Amide product used | g | 14.06 | 11.67 |
| | | mmol | 8.99 | 7.08 |
| | Purified end product | g | 12.5 | 8.8 |
| | | Purity (%) | 100 | 100 |
| | | Yield (%) | 85.5 | 72.4 |

*: improved process on Example 2

Other lipid compounds were also synthesized in the same manner.

Preparation of thin films composed of the compounds:

Example 9 (Preparation of thin films respectively composed of compounds 5, 9, 10, 12, Cf. 1 and Cf. 2)

Each of these compounds was dispersed in a proper solvent to prepare dispersions of 20 mM in concentration for the purpose of obtaining self-supporting cast films having a thickness of about 50 µm. 1 ml of each of the dispersions was cast in a disc shape of 20 mm in diameter, and allowed to stand for vaporization of the solvent.

Table 3 shows results obtained by casting at room temperature using as the solvent water, tetrahydrofuran, ethyleneglycol monomethyl ether, chloroform or perfluorohexane. In Table 3, I stands for water, II tetrahydrofuran, III ethyleneglycol monomethyl ether, IV chloroform, and V perfluorohexane.

Additionally, comparative compound Cf. 1 has the following structure:

Cf. 1

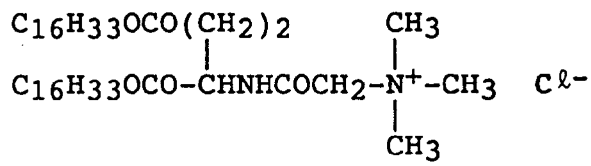

On the other hand, comparative compound Cf. 2 described in Japanese Unexamined Patent No. 58-65,256 has the following structure:

Cf.2

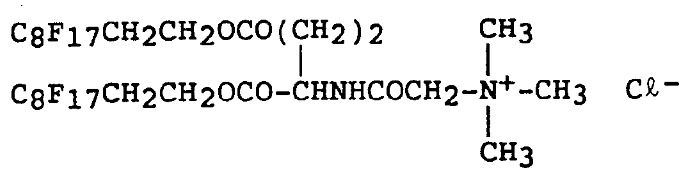

Table 3

Dependence of cast film on dispersing solvent

| Kind of Compound | Kind of Solvent* | Form of Thin Film | X-ray Diffraction Pattern |
|---|---|---|---|
| 5 | I | film | B |
| | II | film | A |
| | III | Film | A |
| | IV | film | A |
| | V | powder | B |
| 9 | I | film | A |
| | II | film | A |
| | III | film | A |
| | IV | film | A |
| | V | film | A |

| Kind of Compound | Kind of Solvent* | Form of Thin Film | X-ray Diffraction Pattern |
|---|---|---|---|
| 10 | I | film | A |
| | II | film | A |
| | III | film | A |
| | IV | film | A |
| | V | film | A |
| 12 | I | film | A |
| | II | film | A |
| | III | film | A |
| | IV | film | A |
| | V | film | A |
| Cf. 1 | I | film | A |
| | II | powder | B |
| | III | powder | B |
| | IV | powder | B |
| | V | powder | B |
| Cf. 2 | I | film | A |
| | II | powder | B |
| | III | fine crystal | B |
| | IV | powder | B |
| | V | powder | B |

Vertical section of the cast film obtained from compound 9 using ethyleneglycol monomethyl ether and that of the cast film obtained from compound 10 using perfluorohexane were viewed by means of a scanning type electron microscope. As a result, they were found to have the structures shown in Figs. 1 and 2 and Figs. 3 and 4, respectively. As is apparent from Figs. 1 to 4, it is seen that ultra-thin bimolecular layers develop in the horizontal direction and are superimposed one over the other to form a macroscopic film.

Regularity of molecular orientation of the cast films was evaluated through X-ray diffraction patterns obtained by irradiating Cu-Ka rays in a parallel direction to the film surface. As a result, there was obtained diffraction pattern A or B which indicates a regular structure as shown in Fig. 5.

In pattern A, a periodical structure of glancing angle region corresponding to the length of about 2 molecules appears in a vertical direction to the film surface, and a 18-degree diffraction corresponding to packing of fluoroalkyl chains appears in a horizontal direction to the film surface. Spacing of the periodical structure was calculated from the diffraction angle of reflection X-ray diffraction profile and Bragg's formula, which coincided

with the measured diffraction pattern. This shows that the diffraction pattern reflects a regular structure analogous to LB layers wherein a bimolecular, multilayer structure develops in a direction parallel to the film surface.

On the other hand, diffraction pattern B appears with powders wherein the bimolecular layer structure does not develop, showing no anisotropy and showing a ring-like, 18-degree diffraction corresponding to packing of the fluoroalkyl chains.

Example 10

A monomolecular layer of compound 7 was prepared as follows according to the LB process.

10 mg of compound 7 was dissolved in 10 ml of a mixed solvent of 4 parts of benzene and 1 part of 2,2,2-trifluoro-ethanol to prepare a solution to spread. 0.150 ml of this solution was spread on pure water kept at 20°C and retained in a Langmuir trough and, after allowing to stand for 10 minutes to evaporate the solvent, then the monomolecular layer thus formed is compressed to a surface pressure of 30 mN/m to obtain a stable monomolecular layer on the surface of water.

The monomolecular layer was transferred onto a clean slide glass by a vertical immersion technique, then dried in a desiccator.

The thus-obtained monomolecular layer showed a contact angle of 111.6 and 97.2 for water and diiodomethane, respectively, measured at room temperature (20°C) according to the liquid drop method using a contact angle meter. The free surface energy of the monomolecular layer ($\gamma_s$) was calculated to be 10.4 erg/cm$^2$. Additionally, the free surface energy ($\gamma_s$) was calculated according to the following formula:

$$\gamma_s = \gamma_s^d + \gamma_s^h$$

$$1 + \cos\theta = 2\sqrt{\gamma_s^d}\left(\frac{\sqrt{\gamma_l^d}}{\gamma_{lv}}\right) + 2\sqrt{\gamma_s^h}\left(\frac{\sqrt{\gamma_l^h}}{\gamma_{lv}}\right)$$

[See D.K. Owens et al; "J. Appl. Polym. Sci.," 13, 1741 (1969).]

Table 4

| Liquid | $\gamma_l^d$ | $\gamma_l^h$ | $\gamma_{lv}$ |
|---|---|---|---|
| $H_2O$ | 21.8 | 51.0 | 72.8 |
| $CH_2I_2$ | 49.5 | 1.3 | 50.8 |

In the above formula, symbols stand for the following meanings:

$\gamma_s^d$ : surface free energy based on hydrogen bond;
$\gamma_s^h$ : surface free energy based on dispersing power;
$\theta$ : contact angle;
$\gamma_1^v$ : free energy at gas-liquid interface;
$\gamma_1^d$ : free energy at gas-liquid interface based on hydrogen bond;
$\gamma_1^h$ : free energy at gas-liquid interface based on dispersing power.

LB monomolecular layers were formed in the same manner using the compounds shown in Table 5. Surface characteristics of the resulting monomolecular layers were measured. The results are also shown in Table 5. Additionally, unit of contact angle, $\theta$, is (°), and unit of surface free energy, $\gamma_s$, is (erg/cm$_2$). Comparative compound Cf. 3 has the following chemical structure and is described in Japanese Unexamined Patent Publication No. 58-65256.

Cf. 3

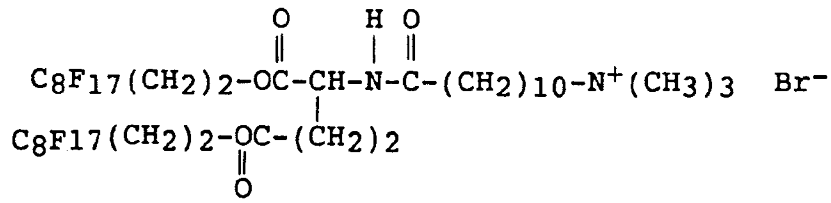

Table 5

Surface characteristics of various LB monomolecular layers

| Compound | Contact Angle, $\theta$ | | Surface Free Energy, $\gamma_s$ |
|---|---|---|---|
| | $H_2O$ | $CH_2I_2$ | |
| 7 | 111.6 | 97.2 | 10.4 |
| 12 | 113.9 | 95.7 | 10.6 |
| 2 | 109.1 | 97.1 | 10.8 |
| 17 | 112.0 | 93.0 | 11.7 |
| 14 | 107.3 | 93.4 | 12.2 |
| 6 | 104.1 | 95.1 | 12.5 |
| 4 | 104.3 | 93.2 | 13.0 |
| Cf.2 | 79.2 | 91.3 | 25.7 |
| Cf.3 | 76.0 | 90.0 | 28.1 |

As is clear from Table 5, the compounds in accordance with the present invention form monomolecular layers having extremely high water- and oil-repelling properties and low surface free energies. This is attributed to that the compounds form films with high molecular orientation and no defects because of the high self-organizing ability of forming molecular layers.

On the other hand, monomolecular layers formed of comparative compounds Cf. 2 and Cf. 3 show much smaller contact angles for water, and are inferior to the monomolecular layers of the compounds in accordance with the present invention in surface characteristics such as water-repelling ability. These comparative compounds are structurally different from compounds 1 and 2 only in that the hydrocarbon chain of the comparative compounds between the fluorocarbon chain and the oxygen atom is sorter and therefore more rigid than that of compounds 1 and 2. Therefore, it is clear that the excellent properties of the monolayers of the present invention originate from the long hydrocarbon chain introduced between the fluorocarbon chain and the connector moiety.

Example 11

Accumulated layers of compound 7 were formed as follows according to the LB process.

A stable monomolecular layer was formed in the same manner as in Example 10. Thereafter, Y-shaped

five monomolecular layers were superimposed on a clean slide glass according to the vertical immersion process, then dried in a desiccator.

The contact angles of the accumulated layers were measured at room temperature (20°C) in the same manner as in Example 10, and were measured to be 113.9 and 100.0 for water and diiodomethane, respectively. The surface free energy, $\gamma_s$, was measured to be 9.2 erg/cm$^2$.

Example 12 (Cast film)

A cast film of compound 6 was prepared in the following manner.

Compound 6 was dispersed in water to prepare a 20 mM dispersion thereof. 1 ml of this dispersion was cast in a disc form of 20 mm in diameter, then allowed to stand to evaporate the solvent to obtain a film of about 50 μm in thickness.

The thus-obtained cast film was subjected to measurement of contact angle in the same manner as in Example 2, and was found to show a contact angle of 118.7 for water and 98.8 for diiodomethane. The surface free energy, $\gamma_s$, was calculated to be 9.2 erg/cm$^2$.

LB monomolecular layers were prepared in the same manner using compounds shown in Table 6. Surface characteristics of the obtained layers were measured, and the results are also shown in Table 6. Additionally, unit of contact angle, θ, is (°), and unit of surface free energy, $\gamma_s$, is (erg/cm$^2$). Comparative compounds Cf. 4 and 5 have the following chemical structures and are described in Japanese Unexamined Patent Publication No. 58-65256.

Cf. 4

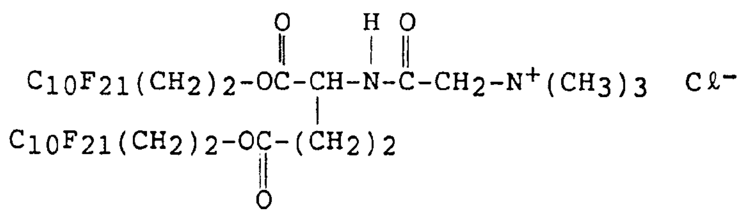

Cf. 5

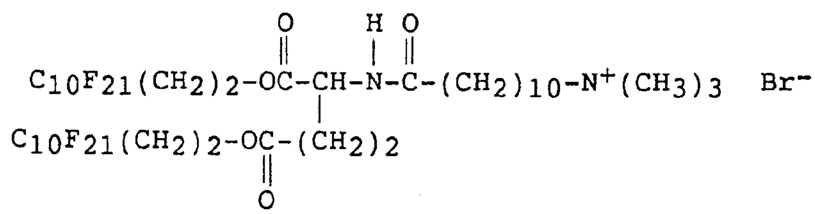

Table 6

Surface characteristics of various cast layers

| Compound | Contact Angle, θ | | Surface Free Energy, γ |
|---|---|---|---|
| | $H_2O$ | $CH_2I_2$ | |
| 6 | 118.7 | 98.8 | 9.2 |
| 3 | 105.9 | 96.9 | 11.6 |
| 1 | 100.5 | 95.1 | 13.6 |
| Cf.1 | 88.5 | 97.5 | 19.2 |
| Cf.4 | 74.6 | 96.8 | 29.6 |
| Cf.5 | 48.2 | 98.0 | - |

As is clear from Table 6, the cast layers of the compounds in accordance with the present invention showed high water- and oil-repelling properties and low surface free energies. This is attributed to that the compounds form layers with high molecular orientation and no defects because of the high self-organizing ability of forming molecular layers.

On the other hand, cast films formed of comparative compounds Cf. 1, Cf. 4 and Cf. 5 show much smaller contact angles particularly for water, and are inferior to the monomolecular layers of the compounds in accordance with the present invention in surface characteristics such as water-repelling ability. These comparative compounds, Cf. 1 and Cf. 4 are structurally different from compounds 1 and 6 only in that the hydrocarbon chain of the comparative compounds between the fluorocarbon chain and the oxygen atom is shorter and therefore more rigid than that of compounds 1 and 6. Therefore, it is clear that the excellent properties of the monolayers of the present invention originate from the long hydrocarbon chain introduced between the fluorocarbon chain and the connector moiety.

Example 13

A muti-layer film of compound 10 was prepared in the following manner according to the LB process.

10 mg of compound 10 was dissolved in 10 ml of a mixed solvent of 9 parts of benzene and 1 part of ethanol to prepare a solution to spread. 0.150 ml of this solution was spread on a dilute aqueous solution of sodium alginate kept at 20°C and retained in a Langmuir trough and, after allowing to stand for 10 minutes to evaporate the solvent, the monomolecular layer thus formed is compressed to a surface pressure of 30 mN/m to obtain a stable monomolecular layer on the surface of water.

The monomolecular layer was transferred onto a porous polycarbonate film (pore size: 0.015 μm) fixed on a teflon plate according to a vertical immersion technique, thus Z-type 25 layers being superimposed.

Measurement of the gas permeability of the thus-obtained film revealed that it showed an oxygen permeation rate of 1.0 X $10^{-3}$ $cc/cm^2 \cdot sec \cdot cmHg$ for effective pore area, and that selectivity between oxygen and nitrogen is 2.5 in terms of the ratio of oxygen permeation rate/nitrogen permeation rate.

Example 14-16

A monomolecular layer of compound 10 was prepared in the following manner according to the LB process.

A 200-A aluminum layer was formed on a polycarbonate porous film (pore size: 0.015 μm) fixed on a teflon plate by the vacuum deposition process to smoothen the surface.

Then, a stable monomolecular layer of compound 10 was formed on a dilute aqueous solution of sodium

alginate retained in a Langmuir trough. Then a single monomolecular layer of compound 10 was transferred onto the above-described porous film according to a vertical immersion technique.

Measurement of the gas permeability of the thus-obtained monomolecular layer revealed that it shows an oxygen permeation rate of 2.1 X $10^{-2}$ cc/cm$^2$·sec·cmHg for effective pore area, and that selectivity between oxygen and nitrogen is 2.5 is terms of the ratio of oxygen permeation rate/nitrogen permeation rate.

LB monomolecular layers were prepared in the same manner using the compounds shown in Table 7. Gas-separating ability of each layer was measured. Results of the measurement are shown in Table 7 together with results for comparative sample Cf using carboxymethyl cellulose as additional component. Additionally, in Table 7, oxygen permeation rate, $Ro_2$, is represented as a value obtained by measurement at 25°C [cc/cm$^2$·sec·cmHg X $10^{-3}$] and selectivity as an oxygen permeation rate-to nitrogen permeation rate. PSS and CMC in Table 7 stand for polystyrenesulfonic acid and carboxymethylcellulose, respectively.

Table 7

Various LB layers and gas-separating abilities thereof

| Example No. | Layer-forming components | | Separation Abilities | |
|---|---|---|---|---|
| | Lipid | Additional Component | $Ro_2$ | Selectivity |
| 14 | Compound 10 | Alginic acid | 21 | 3.2 |
| 15 | Compound 10 | - | 6.6 | 2.9 |
| 16 | Compound Cf.2 | PSS | 18 | 3.2 |
| Cf | Compound Cf.2 | CMC | 2900 | (1.0) |

As is clear from Table 7, the LB monomolecular layers of the Example 14-16, which have an extremely thin thickness, show an extremely high oxygen permeation rate about 20 times as high as that obtained in Example 13. Table 7 also shows excellent selectivity of the layers, which means that the layers have almost no defects such as pinholes despite the monomolecular structure thereof. Such advantages originate from the high self-organizing, molecular layer-forming ability of the compounds used.

Compound 10 used in Example 15 has an enough flexibility and improved molecular orientation properties because of introduction of an enough long hydrocarbon chain and a double bond, and hence a monomolecular layer having good permeation properties is obtained in Example 15 without adding any additional component or additive.

On the other hand, compound Cf. 2 used in Comparative Example Cf has only a short hydrocarbon chain and, in some cases, it shows no selectivity as in Comparative Example Cf. This is attributed to that the monomolecular layer has many defects due to too high crystallinity of the compound used.

However, in case where the compound Cf. 2 is used in combination with polystyrenesulfonic acid, crystallinity of the layer is surmised to decrease based on the expansion behavior of the monomolecular layer formed on the surface of water. Results of Example 16 support this surmise. That is, the monomolecular layer obtained in Example 16 has good permeation properties despite that the lipid compound used has introduced thereinto only a short hydrocarbon chain.

Additionally, multi-layer films were prepared in the same manner as in Example 13 using the combination of the Comparative Example. Gas permeability of the films was measured to obtain the following results. That is, a 20-layer film showed no selectivity between oxygen and nitrogen, whereas a 50-layer film showed a selectivity of 1.8.

Example 17

10 mg of compound Cf. 5, a 1/2 equivalent amount of salcomine and a 1/2 equivalent amount of polyvinylpyridine were dissolved in 10 ml of a mixed solvent of 3 parts of benzene and 2 parts of methanol to prepare

a solution to spread. 0.15 ml of this solution was spread on a dilute aqueous solution of carboxymethylcellulose and, after leaving for 10 minutes to evaporate the solvent, the formed monomolecular layer was compressed to a surface pressure of 30 mN/m to form a stable monomolecular layer on the surface of water.

Thereafter, Y-type 20 layer were formed in the same manner as in Example 14 according to the vertical immersion process. Measurement of the gas permeability of the thus-obtained layers revealed that the layers showed an oxygen permeation rate of 9.1 X $10^{-4}$ cc/cm$^2$·sec·cmHg for effective pore area, and that selectivity between oxygen and nitrogen is 2.7 in terms of the ratio of oxygen permeation rate/nitrogen permeation rate.

A single monomolecular layer was formed in the same manner as in Example 14-16 according to the vertical immersion process. Measurement of the gas permeability of the thus-obtained layer revealed that it showed an oxygen permeation rate of 1.9 X $10^{-2}$ cc/cm$^2$·sec·cmHg for effective pore area, and that selectivity between oxygen and nitrogen is 1.8 in terms of the ratio of oxygen permeation rate/nitrogen permeation rate.

Example 18 (adsorption-process monomolecular layer)

A monomolecular layer of compound 10 was formed in the following manner according to the adsorption process.

A 200-Å aluminum layer was formed on a polycarbonate porous film (pore size: 0.015 μm) fixed on a teflon plate by the vacuum deposition process to smoothen the surface. Then, 1 mg of compound 10 was dissolved in 5 ml of a mixed solvent of 9 parts of hexadecane and 1 part of 1, 1, 2-trichloro-1, 2, 2-trifluoroethane to prepare a dilute solution. The above-described porous film was immersed in this solution for one minute, raised therefrom, then dried at 80°C for 30 minutes.

Measurement of the gas permeability at 20°C of the thus-obtained monomolecular layer revealed that it shows an oxygen permeation rate of 1.6 X $10^{-2}$ cc/cm$^2$·sec·cmHg for effective pore area, and that selectivity between oxygen and nitrogen is 2.7 in terms of the ratio of oxygen permeation rate/nitrogen permeation rate.

As has been described hereinbefore, the synthetic lipids of the present invention, which possess both a fluorocarbon chain excellent in hydrophobicity and a hydrocarbon chain excellent in orientation properties as the hydrophobic moiety, provide a wide choice of organic solvents as solvents for dispersing them in addition to water.

The polyfluoro compounds show regularly orienting properties and form molecular layers having extremely small defects on a substrate. Therefore, water- and oil-repelling agents for surface treatment can be obtained utilizing the above-described properties. In addition, the compounds themselves can be used as surface-lubricating agents, releasing agents, etc. Further, the compounds enable one to form a thin film of from monomolecular layer to a film with any thickness up to several ten μm. The thus-formed thin films have a surface with excellent properties. Accordingly, adaptability to a substrate is extremely excellent. Still further, use of the thin layer prepared from the fluoroalkyl compound as a selectively permeating active layer serves to markedly decrease the number of monomolecular layers to accumulate and to provide a substance-separating film having excellent permeation constant and separation constant. Therefore, the thus-obtained substance-separating film is expected to find many applications in various fields as highly functional membranes having excellent properties such as oxygen permeable membranes, solute-extracting membranes, etc.

While the present invention has been described in detail and with reference to specific embodiments thereof, it is apparent to one skilled in the art that various changes and modifications can be made therein without departing from the spirit and the scope of the present invention.

## Claims

1. Polyfluoroalkyl compounds represented by the following general formula:

$$(R\text{-}X\text{-})_2\text{-}Q\text{-}Y$$

wherein

R represents $CF_3(CF_2)_n$- (n: an integer of 7 to 13),

X represents a hydrocarbyl linking group containing 6 to 11 carbon atoms and optionally containing a double bond, triple bond or ether bond at any position,

Q represents a connector between the hydrophobic group of R-X- and the hydrophilic group of Y, and

Y represents a hydrophilic group.

2. Polyfluoroalkyl compounds as claimed in claim 1 and represented by the following general formula:

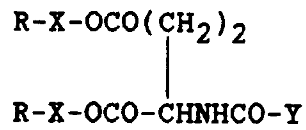

wherein R, X and Y are as defined in claim 1.

3. A thin film having a low surface activity, which is prepared from at least one polyfluoroalkyl compound as claimed in claim 1 or 2.

4. A substance-separating film using a compound layer or layers having regular molecular orientation, which comprises a porous support having supported thereon a selectively permeable compound layer or layers prepared from at least one polyfluoroalkyl compound represented by the following general formula:

$$(R\text{-}X\text{-})_2\text{-}Q\text{-}Y$$

wherein

R represents $CF_3(CF_2)_n$- (n: an integer of 7 to 13),

X represents a hydrocarbyl linking group containing 1 to 11 carbon atoms and optionally containing a double bond, triple bond or ether bond at any position,

Q represents a connector between the hydrophobic group of R-X- and the hydrophilic group of Y, and

Y represents a hydrophilic group.

5. A substance-separating film using a compound layer or layers having regular molecular orientation, which comprises a porous support having supported thereon a selectively permeable compound layer or layers prepared from at least one polyfluoroalkyl compound represented by the following general formula:

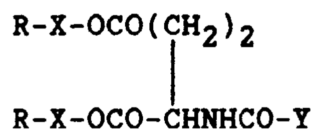

wherein

R represents $CF_3(CF_2)_n$- (n: an integer of 7 to 13),

X represents a hydrocarbyl linking group containing 1 to 11 carbon atoms and optionally containing a double bond, triple bond or ether bond at any position, and

Y represents a hydrophilic group.

6. A substance-separating film as claimed in claim 4 or 5 wherein the hydrocarbyl linking group X contains at least 6 carbon atoms.

7. A process for forming a compound layer having regular molecular orientation, which comprises dispersing in a solvent at least one polyfluoroalkyl compound as claimed in claim 1 or 2, to prepare a solution to spread, then casting the solution in a film-like state.

8. A process for preparing a polyfluoroalkyl compound represented by the following general formula (1):

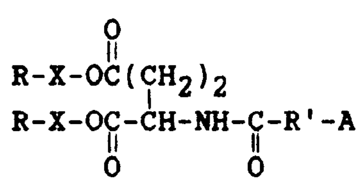

(1)

(wherein

R represents $CF_3(CF_2)_n$- (n: an integer of 7 to 13),

X represents a hydrocarbyl linking group containing 6 to 11 carbon atoms and optionally containing a double bond, triple bond or ether bond at any position,

R′ represents a hydrocarbyl group containing 1 to 12 carbon atoms and optionally containing -

$C_6H_4O$-, and

A represents a quaternary ammonium salt, which comprises subjecting R-X-OH and L-glutamic acid to azeotropic dehydration in the presence of a salt-forming agent, AH to obtain a compound of formula (2):

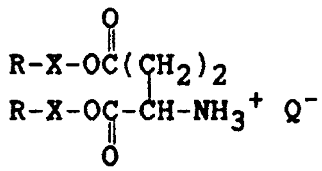

(2)

and subsequently conducting acylation reaction and quaternisation reaction of the compound (2), said acylation being conducted by reacting the compound (2) with an active derivative of ω-halocarboxylic acid after removal of the salt-forming agent with an alkali carbonate in a hydrophobic solvent, and said quaternisation reaction of the acylation product being conducted using a tertiary amine after purifying the acylation product by bringing it into contact with an alkali carbonate at 50-90°C.

**9.** A process as claimed in claim 8 wherein in the salt forming agent AH, A represents p-toluenesulfonate ion and/or sulfate ion.

**10.** A process as claim in claim 8 or 9, wherein said quaternisation reaction is conducted at 40-90°C and increased pressure for 3-10 hours in the presence of a tertiary amine.

FIG. 1

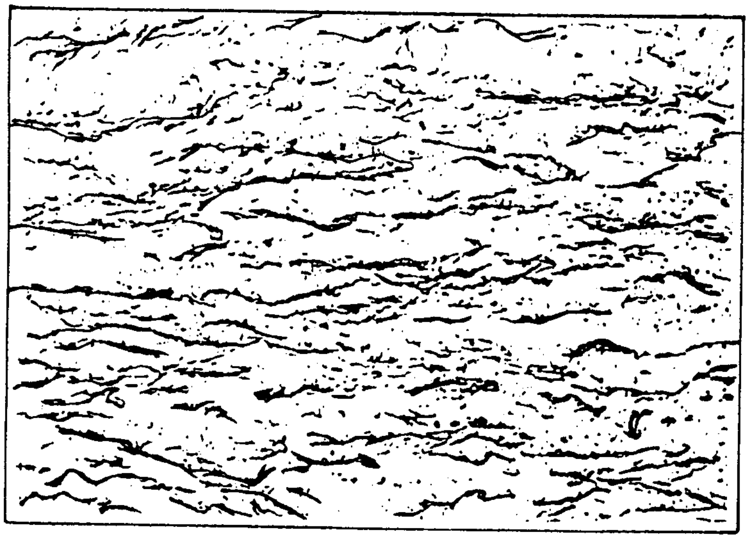

FIG. 2

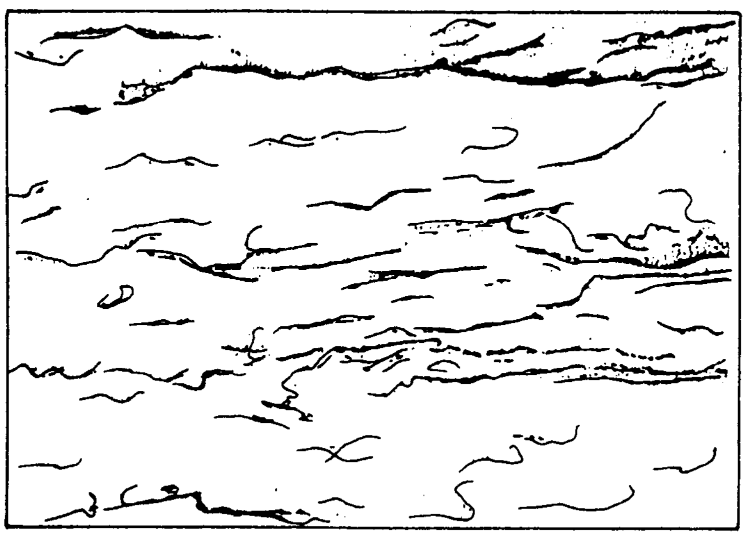

FIG. 3

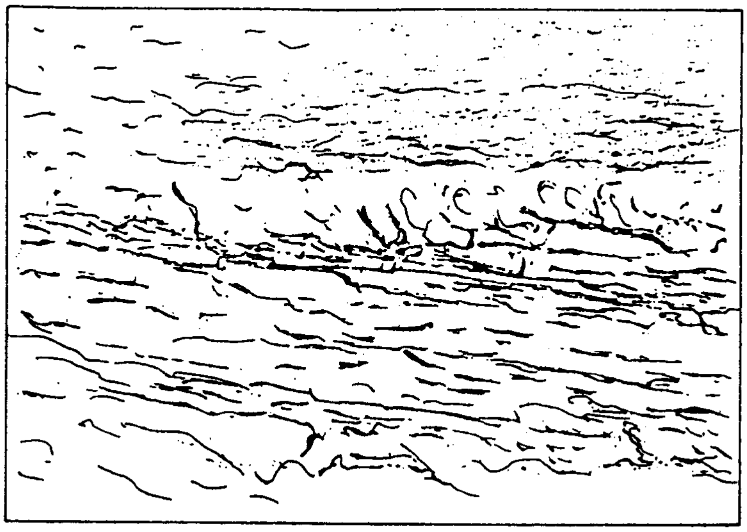

FIG. 4

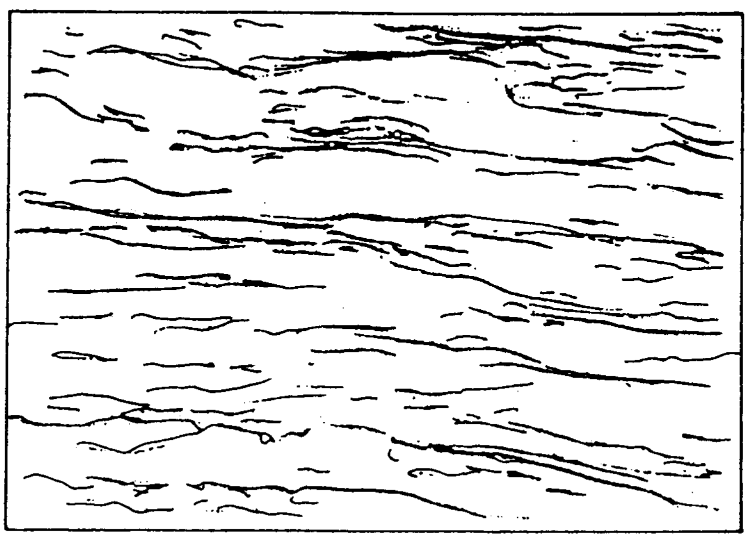

FIG. 5(a)

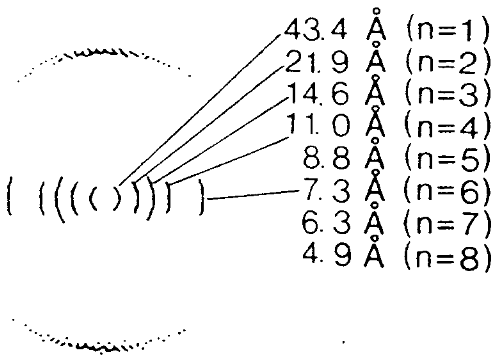


FIG. 5(b)

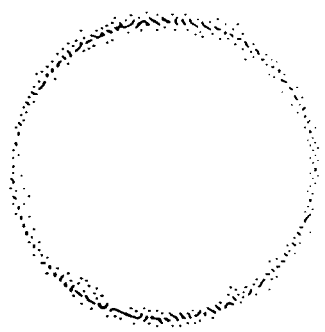

# FIG. 6(a)

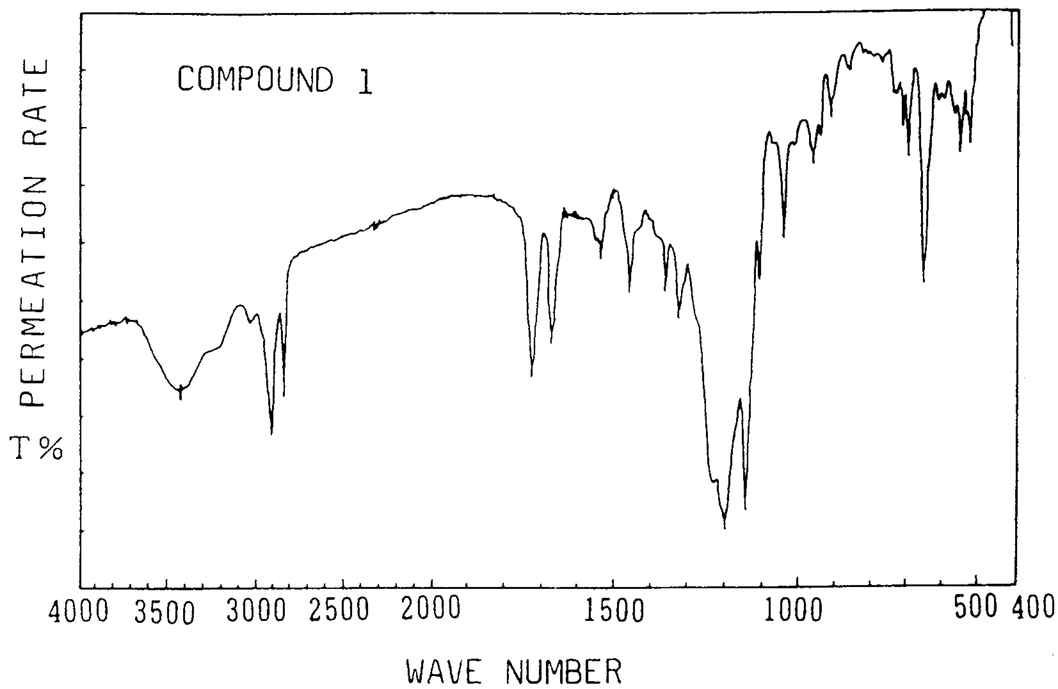
COMPOUND 1
PERMEATION RATE
T%
4000 3500 3000 2500 2000 1500 1000 500 400
WAVE NUMBER

# FIG. 6(b)

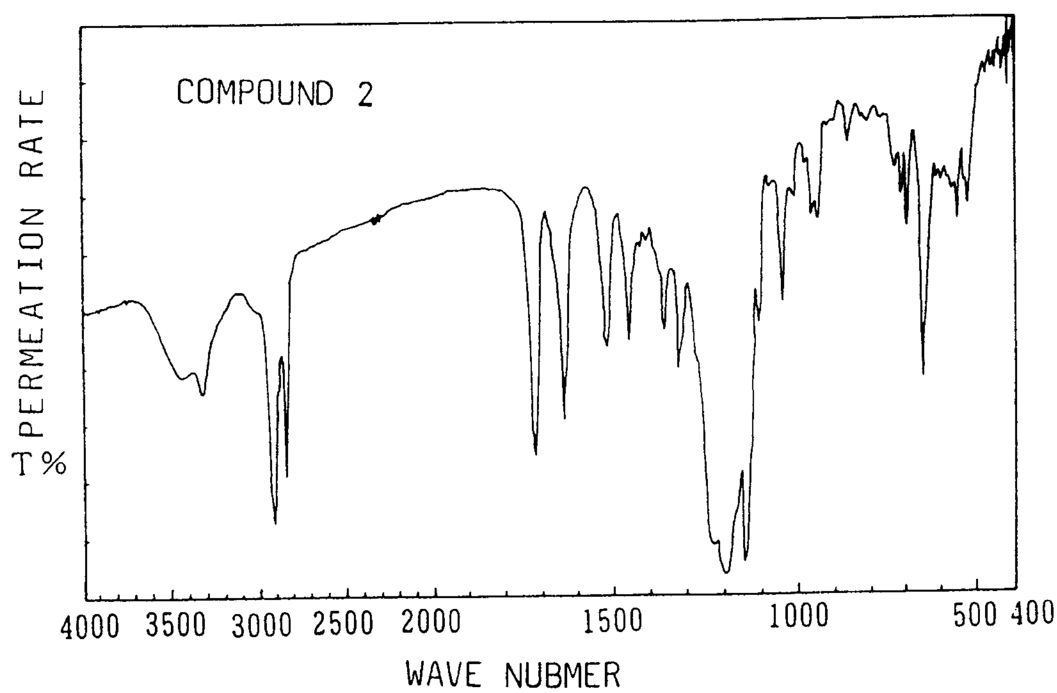
COMPOUND 2
PERMEATION RATE
T%
4000 3500 3000 2500 2000 1500 1000 500 400
WAVE NUBMER

FIG. 6(c)

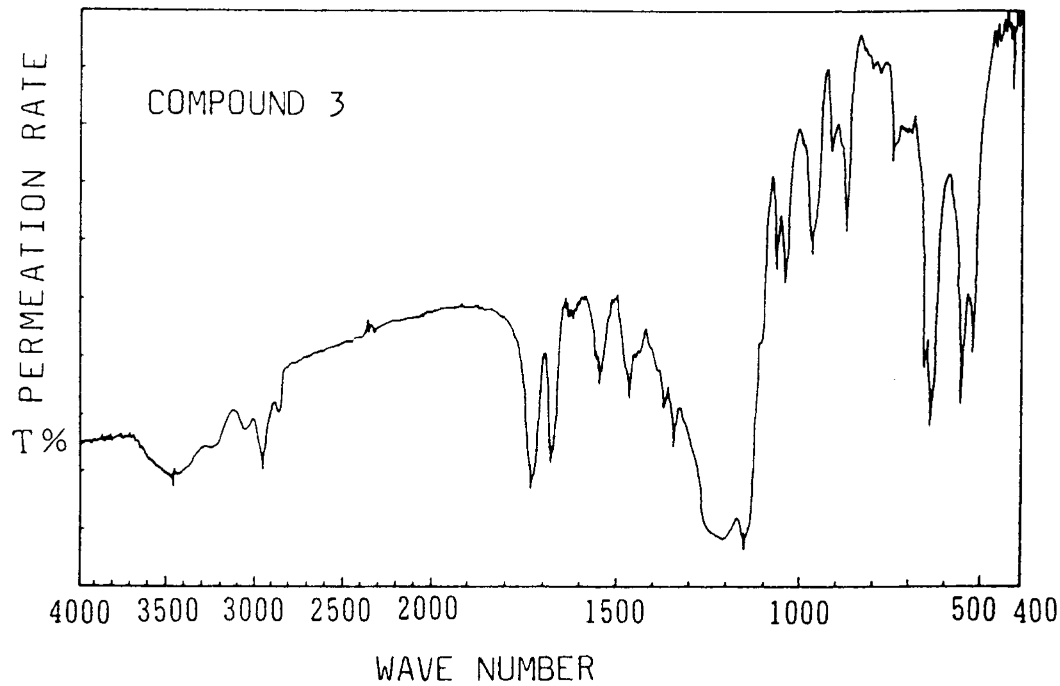
COMPOUND 3
T% PERMEATION RATE
4000 3500 3000 2500 2000 1500 1000 500 400
WAVE NUMBER

FIG. 6(d)

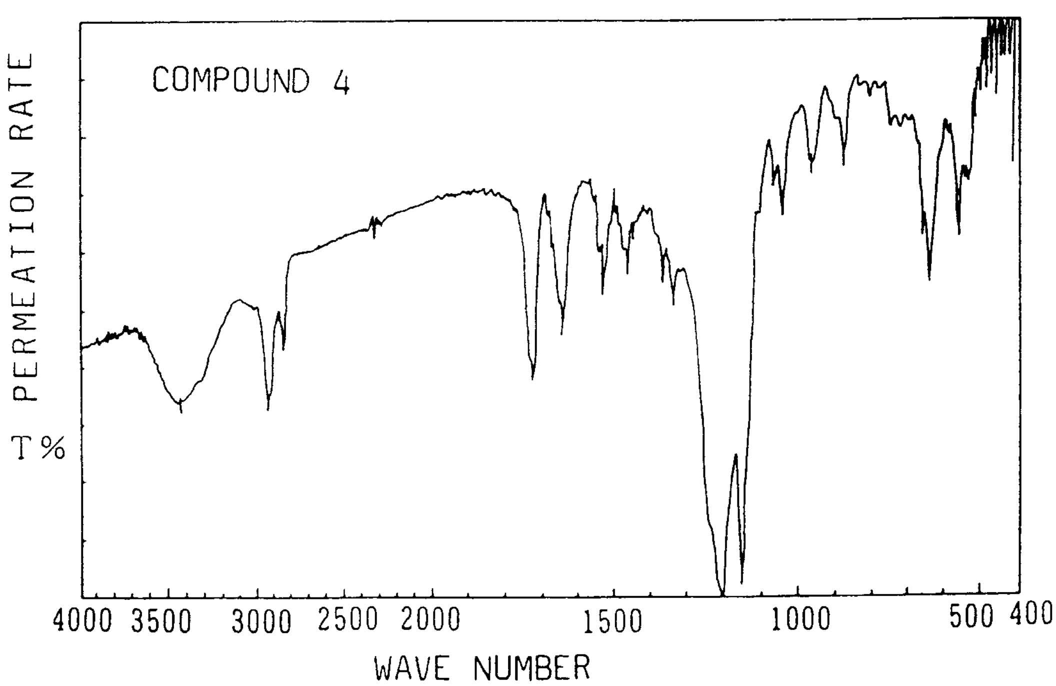
COMPOUND 4
T% PERMEATION RATE
4000 3500 3000 2500 2000 1500 1000 500 400
WAVE NUMBER

FIG. 6(e)

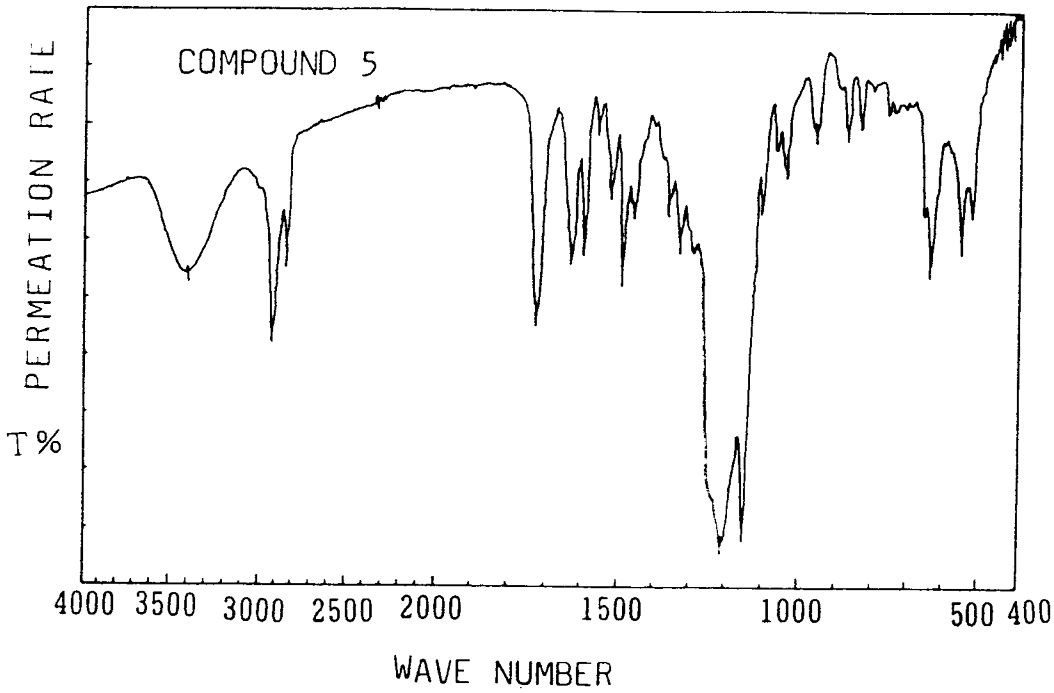
COMPOUND 5
PERMEATION RATE
T %
4000 3500 3000 2500 2000 1500 1000 500 400
WAVE NUMBER

FIG. 6(f)

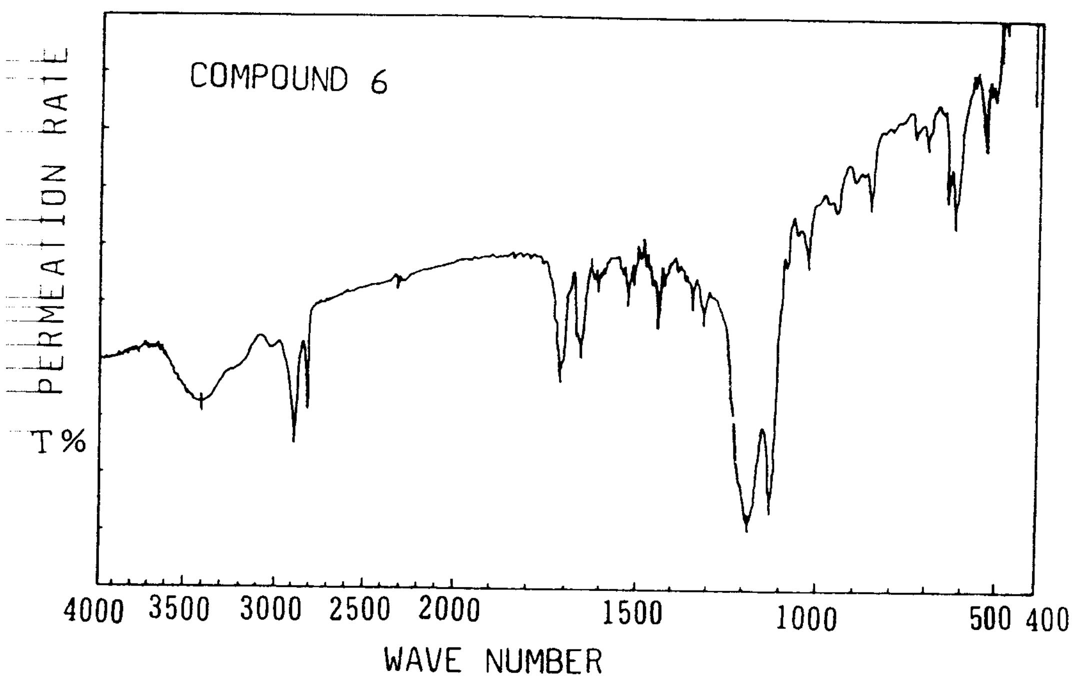
COMPOUND 6
PERMEATION RATE
T %
4000 3500 3000 2500 2000 1500 1000 500 400
WAVE NUMBER

# FIG. 6(g)

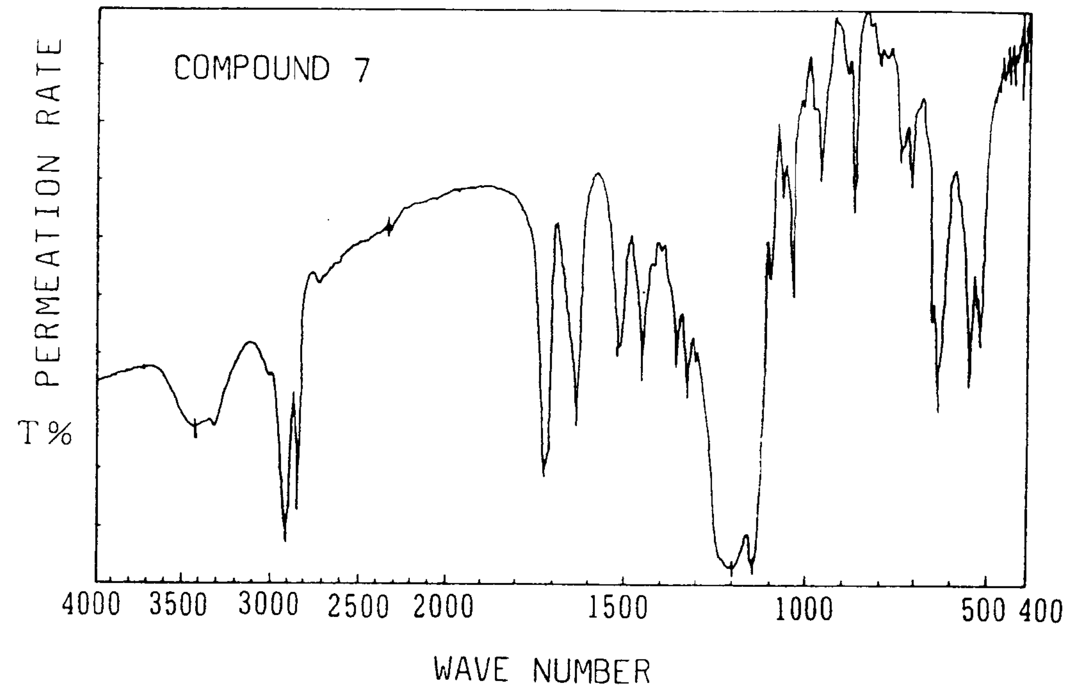
COMPOUND 7
PERMEATION RATE
T %
4000 3500 3000 2500 2000 1500 1000 500 400
WAVE NUMBER

# FIG. 6(h)

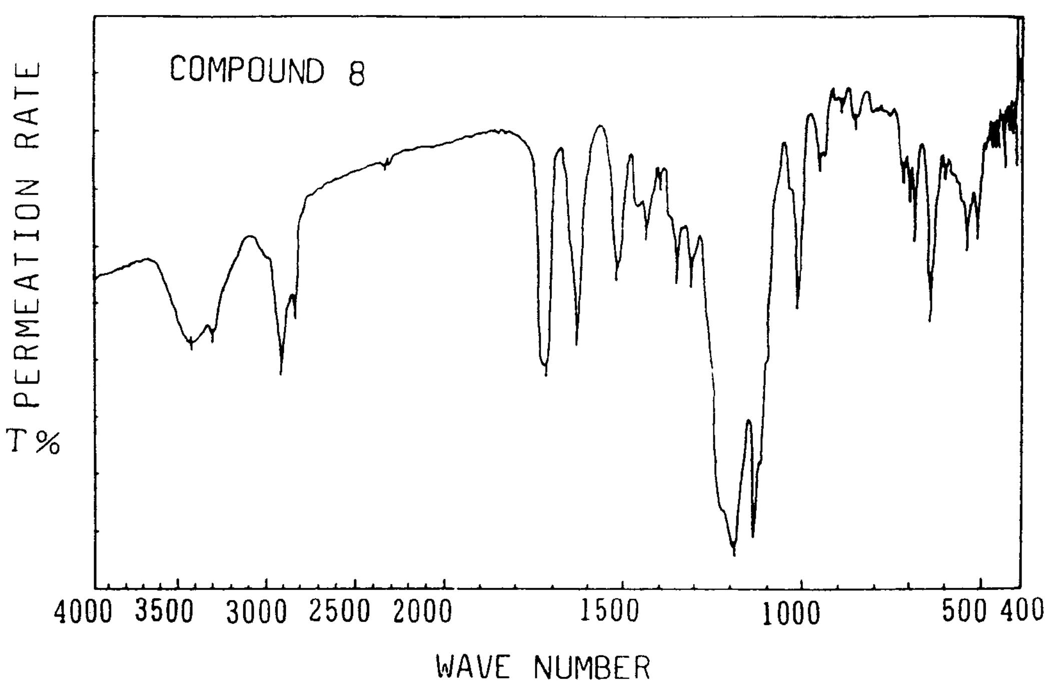
COMPOUND 8
PERMEATION RATE
T %
4000 3500 3000 2500 2000 1500 1000 500 400
WAVE NUMBER

# FIG. 6(i)

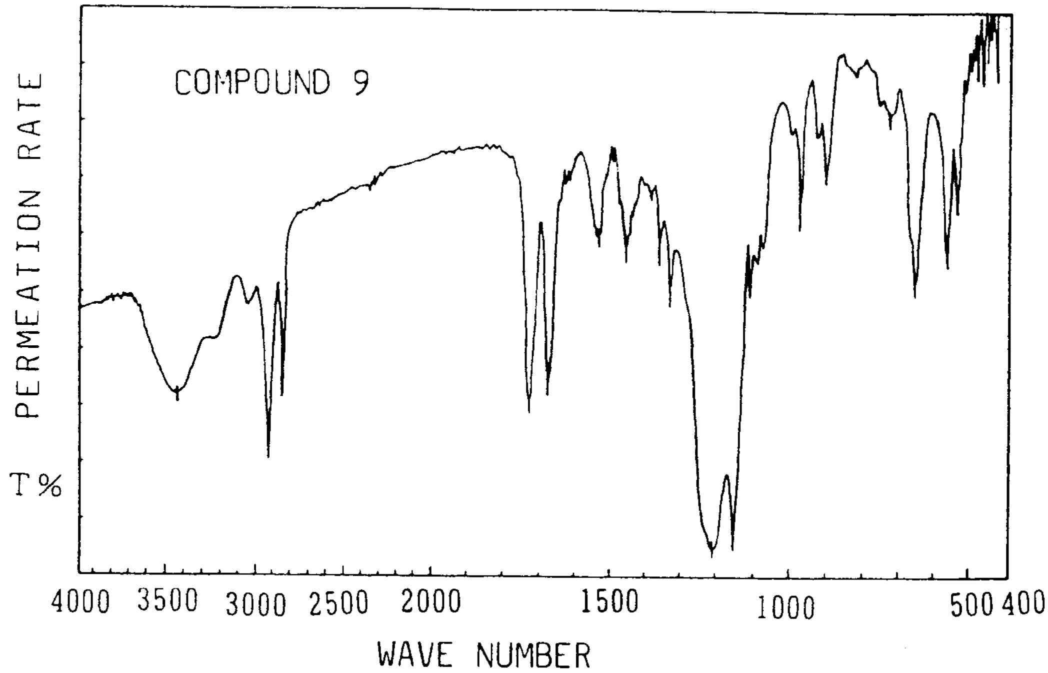
COMPOUND 9
PERMEATION RATE
T %
4000 3500 3000 2500 2000 1500 1000 500 400
WAVE NUMBER

# FIG. 6(j)

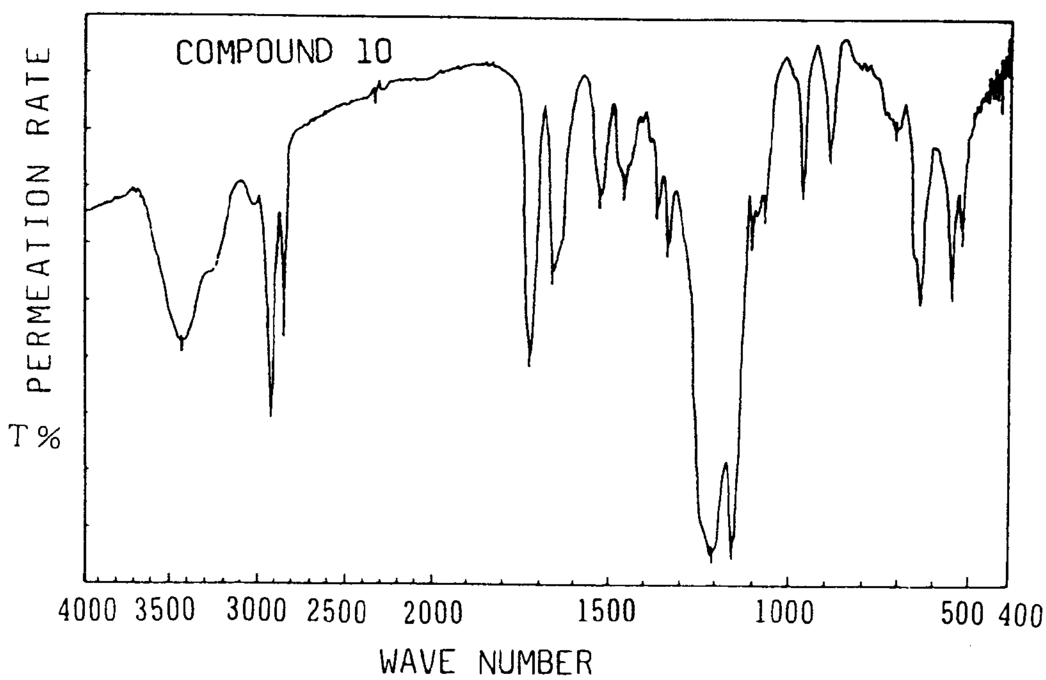
COMPOUND 10
PERMEATION RATE
T %
4000 3500 3000 2500 2000 1500 1000 500 400
WAVE NUMBER

# FIG. 6(k)

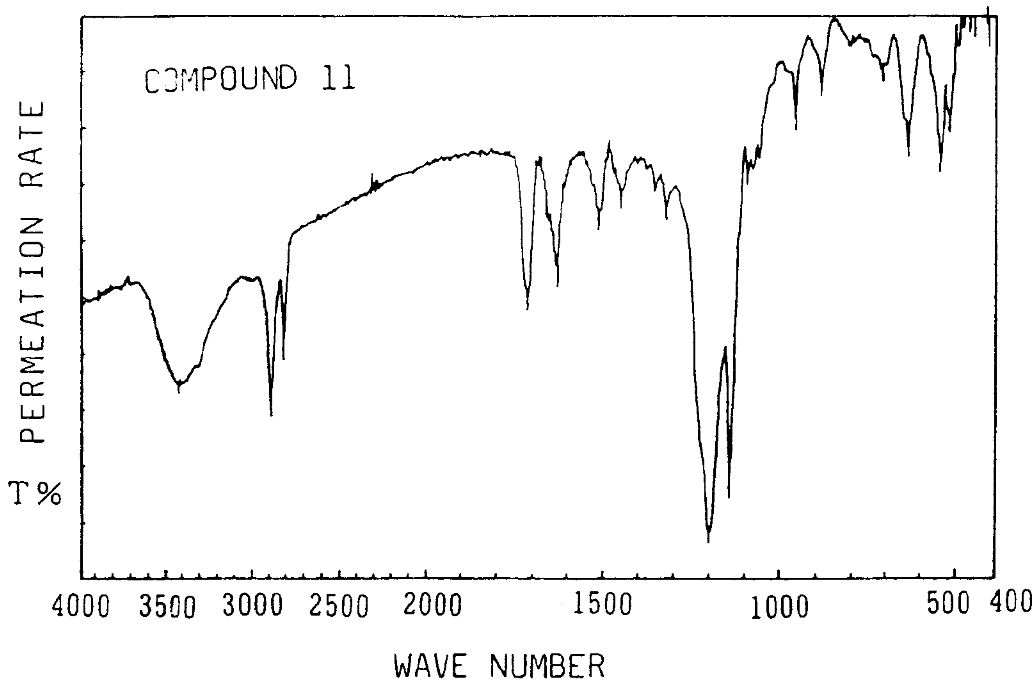
COMPOUND 11
PERMEATION RATE
T%
4000 3500 3000 2500 2000 1500 1000 500 400
WAVE NUMBER

# FIG. 6(l)

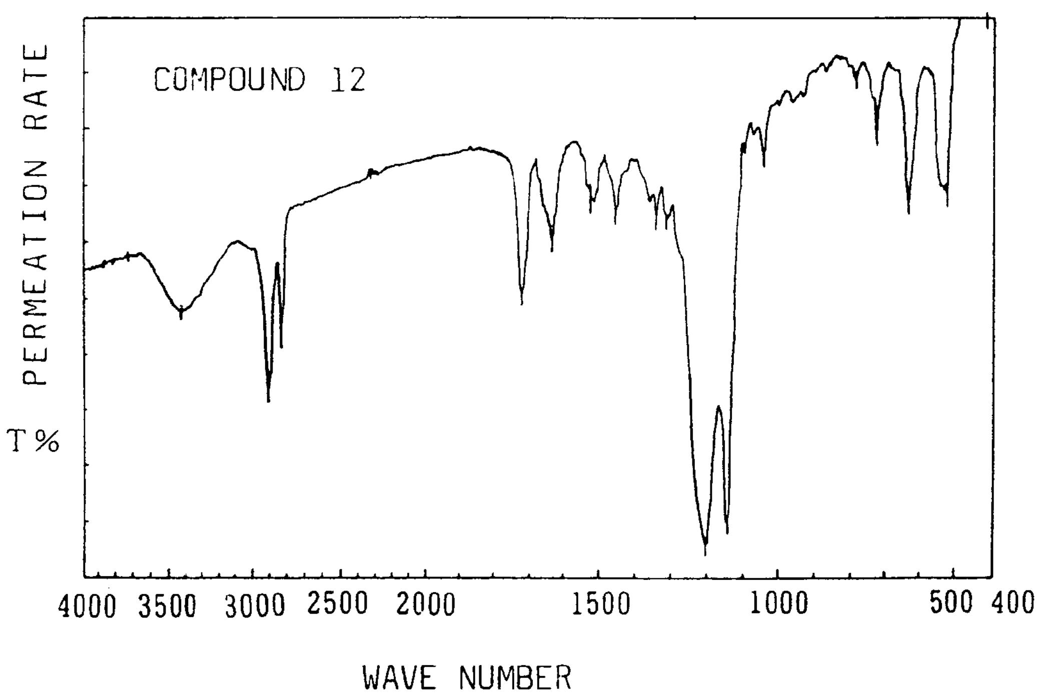
COMPOUND 12
PERMEATION RATE
T%
4000 3500 3000 2500 2000 1500 1000 500 400
WAVE NUMBER

FIG. 6(m)

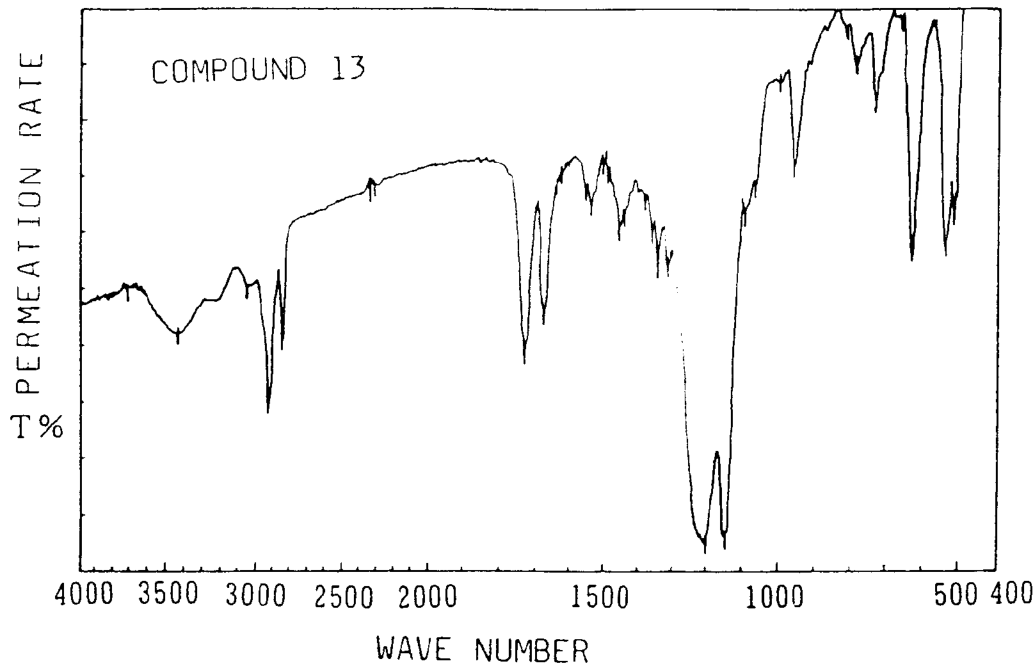
COMPOUND 13
T% PERMEATION RATE
4000 3500 3000 2500 2000 1500 1000 500 400
WAVE NUMBER

FIG. 6(n)

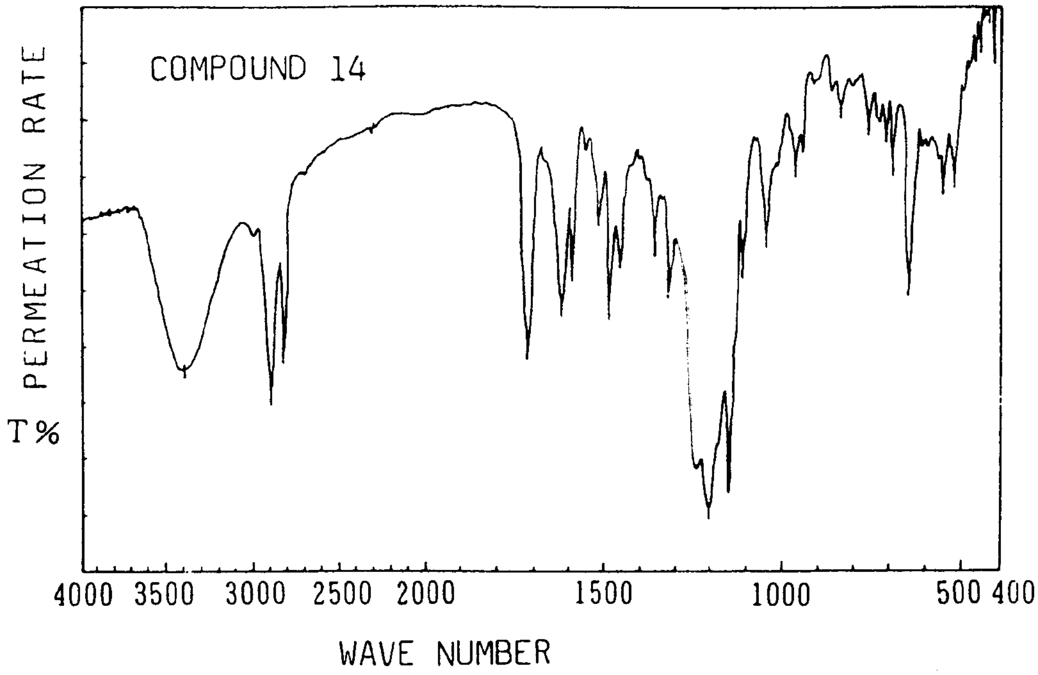
COMPOUND 14
T% PERMEATION RATE
4000 3500 3000 2500 2000 1500 1000 500 400
WAVE NUMBER

FIG. 6(o)

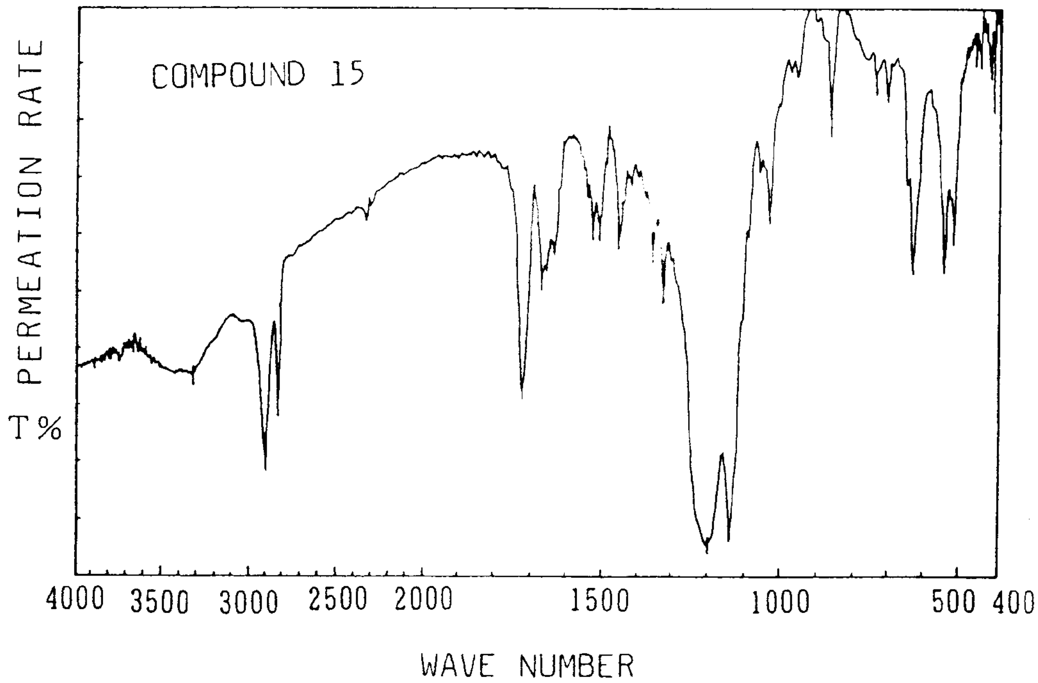
COMPOUND 15
PERMEATION RATE
T%
4000
3500
3000
2500
2000
1500
1000
500
400
WAVE NUMBER

FIG. 6(p)

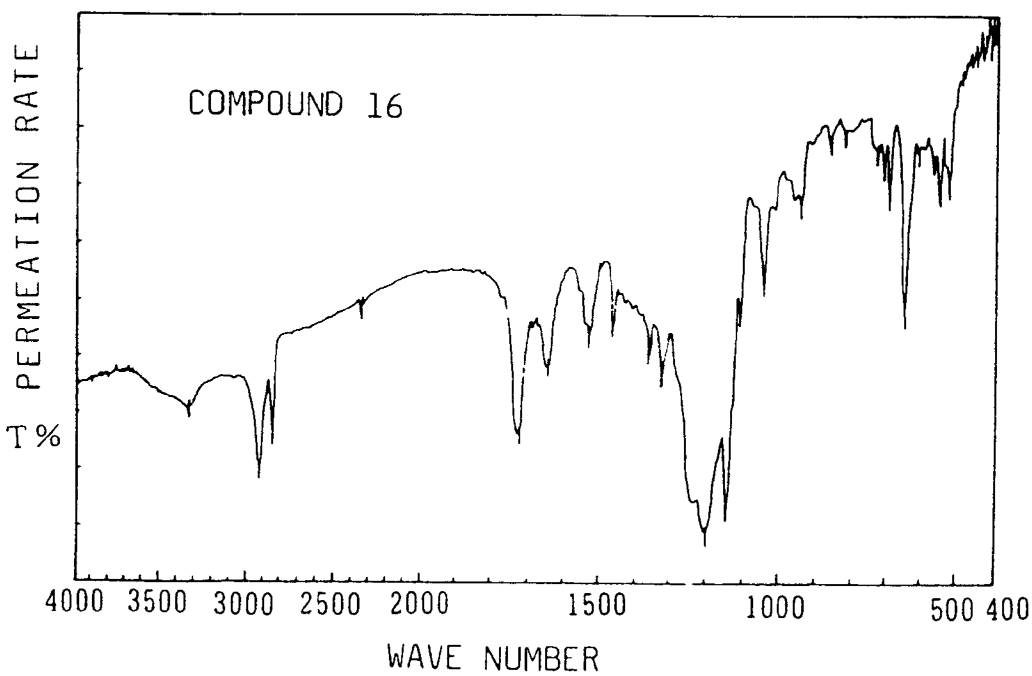
COMPOUND 16
PERMEATION RATE
T%
4000
3500
3000
2500
2000
1500
1000
500
400
WAVE NUMBER

FIG. 6(q)

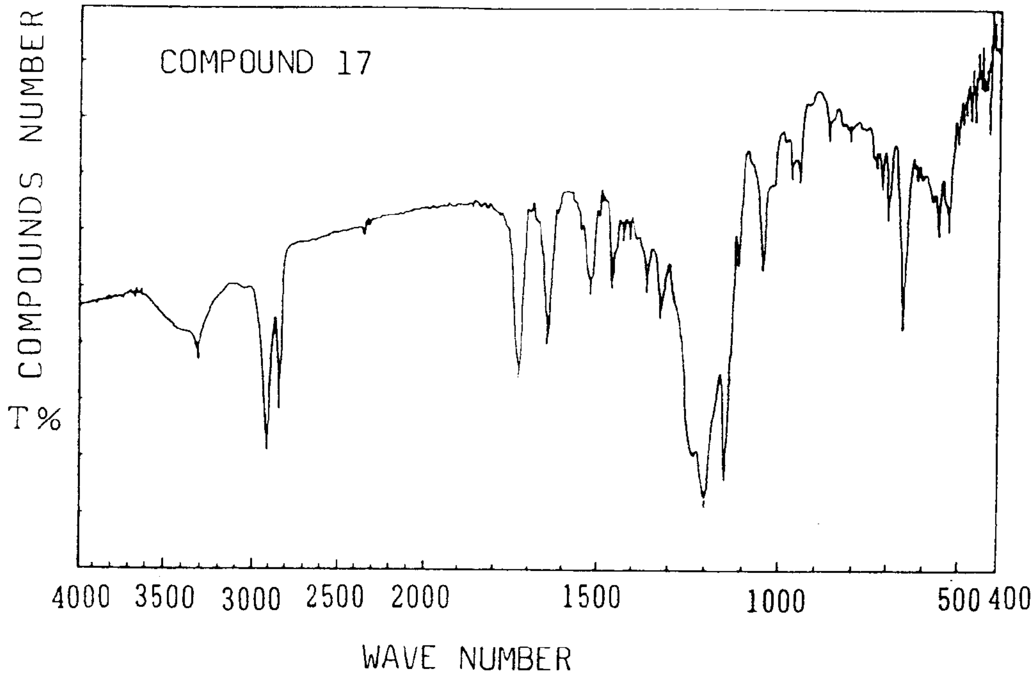
COMPOUND 17
T% COMPOUNDS NUMBER
4000 3500 3000 2500 2000 1500 1000 500 400
WAVE NUMBER

FIG. 6(r)

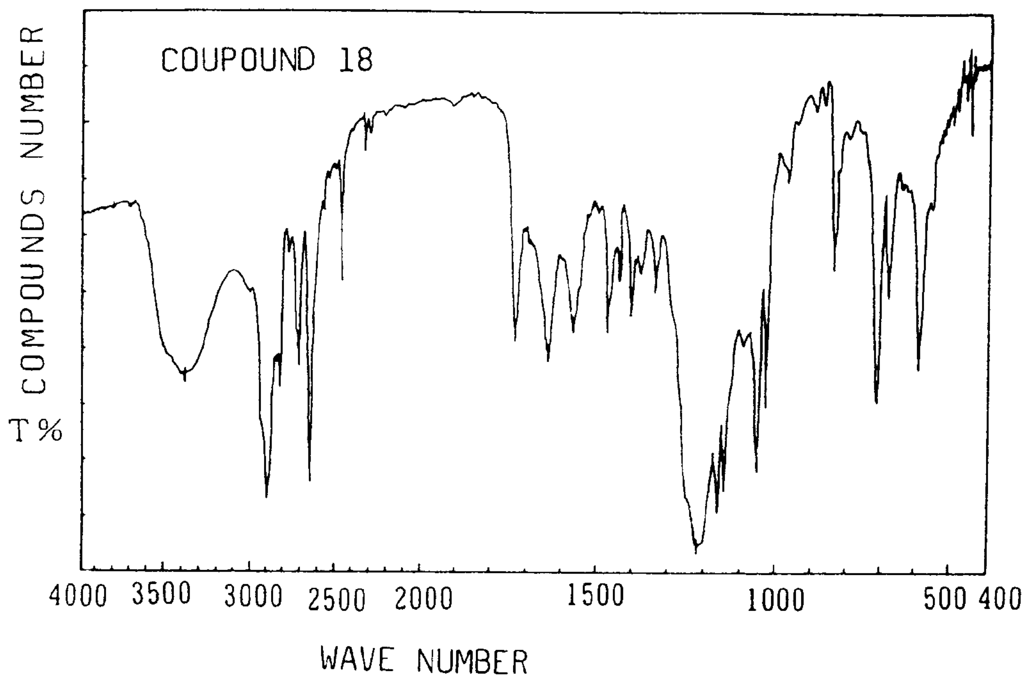
COUPOUND 18
T% COMPOUNDS NUMBER
4000 3500 3000 2500 2000 1500 1000 500 400
WAVE NUMBER

# FIG. 6(s)

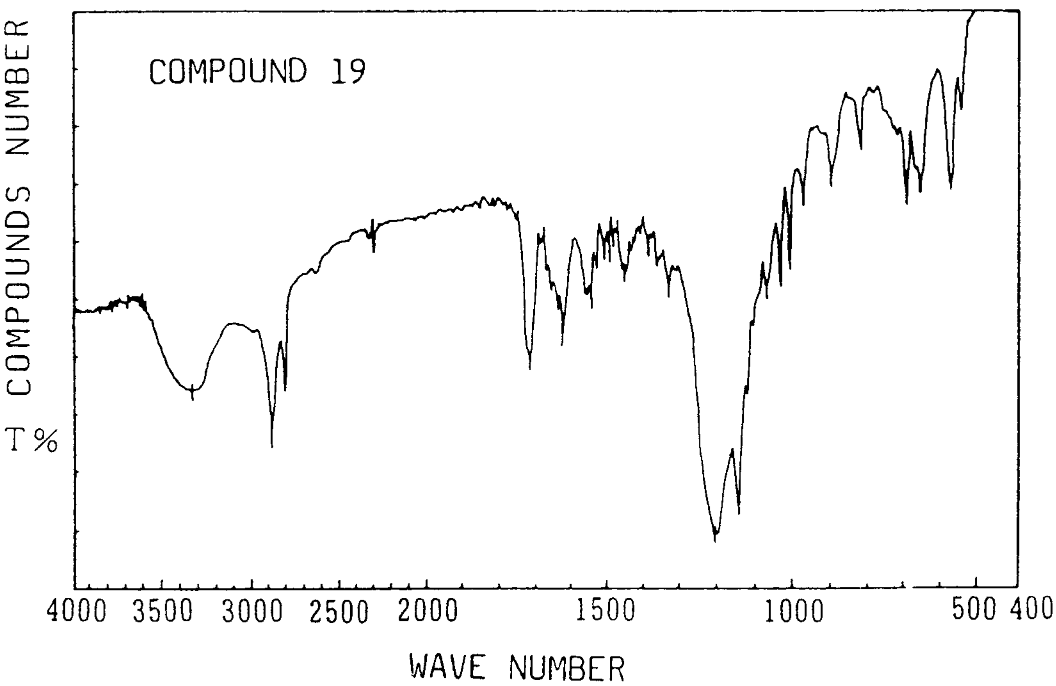
COMPOUND 19
T% COMPOUNDS NUMBER
4000 3500 3000 2500 2000 1500 1000 500 400
WAVE NUMBER

European Patent Office

# EUROPEAN SEARCH REPORT

Application Number

EP 91 30 5991

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| P,X | WO-A-9 015 807 (APPLICATIONS ET TRANSFERTS DE TECHNOLOGIES) * Example 10; figure 1/5, column 2, compound 5; column 3, compound 3 * --- | 1,3 | C 07 C 235/52<br>C 07 C 235/74<br>C 07 C 235/12<br>C 07 C 275/16<br>C 07 C 237/12<br>C 07 F 7/18<br>B 01 D 71/06<br>B 05 D 1/20 |
| X | US-A-4 083 756 (ALLIED CHEMICAL) * Table in columns 5-6, compound 2 * --- | 1,4,6 | |
| X | EP-A-0 085 655 (CIBA-GEIGY) * Examples 7,9,11,13,15 * --- | 4,6 | |
| X | US-A-4 239 915 (R.A. FALK) * Example 26 * --- | 4,6 | |
| X | EP-A-0 376 882 (CIBA-GEIGY) * Examples 1,3,12 * --- | 4,6 | |
| A | FR-A- 311 473 (APPLICATIONS ET TRANSFERTS) * Entire document * --- | 2,7 | TECHNICAL FIELDS SEARCHED (Int. Cl.5) |
| D,A | DERWENT WPIL, ONLINE ABSTRACTS, accession no. 83-50644k, Derwent Publications Ltd, London, GB; & JP-A-58 065 256 (ASAHI GLASS) * Abstract * --- | 1,4,7 | C 07 C 235/00<br>C 07 C 275/00<br>C 07 C 237/00 |
| A | DERWENT WPIL, ONLINE ABSTRACT, accession no. 90-112330, Derwent Publications Ltd, London, GB; & JP-A-2 063 534 (TDK CORP.) * Abstract * --- -/- | 1,4,7 | |

The present search report has been drawn up for all claims

| Place of search | Date of completion of the search | Examiner |
|---|---|---|
| THE HAGUE | 10-10-1991 | WELLS A.G. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)

Page 2

European Patent Office

# EUROPEAN SEARCH REPORT

Application Number

EP 91 30 5991

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| P,A | DERWENT WPIL, ONLINE ABSTRACT, accession no. 91-003707, Derwent Publications Ltd, London, GB; & JP-A-2 280 821 (SANYO ELECTRIC) * Abstract * --- | 1,4,7 | |
| P,A | DERWENT WPIL, ONLINE ABSTRACT, accession no. 90-243622, Derwent Publications Ltd, London, GB; & JP-A-2 170 832 (TDK CORP.) * Abstract * --- | 1,4,7 | |
| A | PATENT ABSTRACTS OF JAPAN, ONLINE ABSTRACTS; & JP-A-2 068 171 (TDK CORP.) * Abstract * ----- | 1,4,7 | |
| | | | TECHNICAL FIELDS SEARCHED (Int. Cl.5) |

The present search report has been drawn up for all claims

| Place of search | Date of completion of the search | Examiner |
|---|---|---|
| THE HAGUE | 10-10-1991 | WELLS A.G. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)